# EUROPÄISCHE PATENTANMELDUNG

(11) **EP 2 039 781 A1**
(43) Veröffentlichungstag der Anmeldung: **25.03.2009**
(21) Anmeldenummer: 08159956.5
(22) Anmeldetag: 08.07.2008
(51) Int. Cl.: C12Q 1/68

(54) **Verdrängungsassay zur Detektion von Nukleinsäureoligomer-Hybridisierungsereignissen**

(30) Priorität: 18.09.2007 DE 102007044664
(71) Anmelder: Fidicula GmbH, 82061 Neuried (DE)
(72) Erfinder: Hartwich, Gerhard, 80639 München (DE)
(74) Vertreter: Zipse Habersack Kritzenberger

(57) **Zusammenfassung**

Beschrieben wird ein Verfahren zur Detektion von Nukleinsäureoligomer-Hybridisierungsereignissen, das die Schritte Bereitstellen einer modifizierten Oberfläche, wobei die Modifikation in der Anbindung wenigstens einer Art von Sonden-Nukleinsäureoligomeren besteht, Bereitstellen wenigstens einer Art von Signal-Nukleinsäureoligomeren, wobei die Signal-Nukleinsäureoligomere mit zumindest einem Detektionslabel modifiziert sind und die Signal-Nukleinsäureoligomere einen zu den Sonden-Nukleinsäureoligomeren komplementären oder weitgehend komplementären Abschnitt besitzen, Bereitstellen einer Probe mit Target-Nukleinsäureoligomeren, Inkontaktbringen einer definierten Menge der Signal-Nukleinsäureoligomere mit der modifizierten Oberfläche, Inkontaktbringen der Probe und der darin enthaltenen Target-Nukleinsäureoligomere mit der modifizierten Oberfläche, Detektion der Signal-Nukleinsäureoligomere und Vergleich der bei der Detektion der Signal-Nukleinsäureoligomere erhaltenen Werte mit Referenzwerten umfasst. Erfindungsgemäß besitzen die Signal-Nukleinsäureoligomere eine größere Anzahl an Basen als die Sonden-Nukleinsäureoligomere und weisen zumindest einen Andockabschnitt auf, wobei der Andockabschnitt keine zu einem Abschnitt der Sonden-Nukleinsäureoligomere komplementäre oder weitgehend komplementäre Struktur aufweist und wobei die Target-Nukleinsäureoligomere einen zu dem Andockabschnitt komplementären oder weitgehend komplementären Abschnitt besitzen.

SEQUENCE LISTING

<110> Fidicula GmbH

<120> Verdrängungsassay zur Detektion von
Nukleinsäureoligomer-Hybridisierungsereignissen

<130> FRI152EP

<140> EP
<141> 2008-07-08

<150> DE 10 2007 044 664.2
<151> 2007-09-18

<160> 7

<170> PatentIn version 3.3

<210> 1
<211> 8
<212> DNA
<213> künstliche Sequenz

<400> 1
gttcaaag 8

<210> 2
<211> 8
<212> DNA
<213> künstliche Sequenz

<400> 2
gttcaaag 8

<210> 3
<211> 8
<212> DNA
<213> künstliche Sequenz

<400> 3
gaaacttg 8

<210> 4
<211> 37
<212> DNA
<213> künstliche Sequenz

<400> 4
cgtatctaca tcttccactt acaccaacat atttatt 37

<210> 5
<211> 56
<212> DNA
<213> künstliche Sequenz

<400> 5
ttggtgtatg tggaagatgt agttacggat gtacataatg tcatgttggt actgcg 56

<210> 6
<211> 38
<212> DNA
<213> künstliche Sequenz

<400> 6
ttggtgtaag tggaagatgt agatacgtca attttttt 38

<210> 7
<211> 27
<212> DNA
<213> künstliche Sequenz

<400> 7
cgtaactaca tcttccacat acaccaa 27

## Beschreibung

### Technisches Gebiet

Die vorliegende Erfindung betrifft ein Verfahren zur Detektion von Nukleinsäureoligomer-Hybridisierungsereignissen.

### Stand der Technik

Aus der WO 03/018834 A2 ist ein Verdrängungsassay zur Detektion von Nukleinsäureoligomer-Hybridisierungsereignissen bekannt, das die Schritte Bereitstellen einer modifizierten Oberfläche, wobei die Modifikation in der Anbindung wenigstens einer Art von Sonden-Nukleinsäureoligomeren besteht, Bereitstellen von Signal-Nukleinsäureoligomeren, Bereitstellen einer Probe mit Target-Nukleinsäureoligomeren, Inkontaktbringen einer definierten Menge der Signal-Nukleinsäureoligomeren mit der modifizierten Oberfläche und Inkontaktbringen der Probe und der darin enthaltenen Target-Nukleinsäureoligomeren mit der modifizierten Oberfläche, Detektion der Signal-Nukleinsäureoligomere, sowie Vergleich der bei der Detektion der Signal-Nukleinsäureoligomeren erhaltenen Werte mit Referenzwerten umfasst.

Als nachteilig an diesem Verfahren hat sich herausgestellt, dass die Verdrängung der an die Sonden-Nukleinsäureoligomere hybridisierten Signal-Nukleinsäureoligomere durch die Target-Nukleinsäureoligomere eine relativ geringe Reaktionsgeschwindigkeit aufweist. Da der Nachweis der Target-Nukleinsäureoligomere bei der weit überwiegenden Zahl der Anwendungen in möglichst kurzer Zeit erfolgen soll, stellt die langsam ablaufende Verdrängung der Signal-Nukleinsäureoligomere durch die Target-Nukleinsäureoligomere einen gravierenden Nachteil des in der WO 03/018834 A2 beschriebenen Verfahrens dar.

### Darstellung der Erfindung

Aufgabe der vorliegenden Erfindung ist es deshalb, ein Verdrängungsassay zur Detektion von Nukleinsäureoligomer-Hybridisierungsereignissen zu schaffen, das den Nachweis der Target-Nukleinsäureoligomere in einem gegenüber dem Stand der Technik verkürzten Zeitraum ermöglicht.

Diese Aufgabe wird durch das Verfahren gemäß unabhängigem Patentanspruch 1 gelöst. Weitere vorteilhafte Details, Aspekte und Ausgestaltungen der vorliegenden Erfindung ergeben sich aus den abhängigen Ansprüchen, der Beschreibung, den Figuren und den Beispielen.

Im Rahmen der vorliegenden Erfindung werden die folgenden Abkürzungen und Begriffe benutzt:
- DNA: Desoxyribonukleinsäure
- RNA: Ribonukleinsäure
- A: Adenin
- G: Guanin
- C: Cytosin
- T: Thymin
- U: Uracil
- Base: A, G, T, C oder U
- Bp: Basenpaar
- Nukleinsäure: Wenigstens zwei kovalent verbundene Nukleotide oder wenigstens zwei kovalent verbundene Pyrimidin- (z.B. Cytosin, Thymin oder Uracil) oder Purin-Basen (z.B. Adenin oder Guanin). Der Begriff Nukleinsäure bezieht sich auf ein beliebiges "Rückgrat" der kovalent verbundenen Pyrimidin- oder Purin-Basen, wie z.B. auf das Zucker-Phosphat Rückgrat der DNA, cDNA oder RNA, auf ein Peptid-Rückgrat der PNA oder auf analoge Strukturen (z.B. Phosphoramid-, Thio-Phosphat- oder Dithio-Phosphat-Rückgrat). Wesentliches Merkmal einer Nukleinsäure ist es, dass sie natürlich vorkommende cDNA oder RNA sequenzspezifisch binden kann.
- Nukleotid, nt: Monomerbaustein eines Nukleinsäureoligomers
- Oligonukleotid, Oligo: Äquivalent zu Nukleinsäureoligomer, also z.B. ein DNA-, PNA- oder RNA-Fragment nicht näher spezifizierter Basenlänge.
- Sequenz: Nukleotidabfolge in einem Nukleinsäureoligomer
- komplementär: Zur Ausbildung der Watson-Crick Struktur doppelsträngiger Nukleinsäureoligomere hybridisieren die beiden Einzelstränge, wobei die Nukleotidabfolge des einen Strangs komplementär zur Nukleotidabfolge des anderen Strangs ist, so dass die Base A (bzw. C) des einen Strangs mit der Base T (bzw. G) des anderen Strangs Wasserstoffbrücken ausbildet (bei RNA ist T durch Uracil ersetzt).
- Mismatch: Zur Ausbildung der Watson-Crick Struktur doppelsträngiger Nukleinsäureoligomere hybridisieren die beiden Einzelstränge derart, dass die Base A (bzw. C) des einen Strangs mit der Base T (bzw. G) des anderen Strangs Wasserstoffbrücken ausbildet (bei RNA ist T durch Uracil ersetzt). Jede andere Basenpaarung innerhalb des Hybrids bildet keine Wasserstoffbrücken aus, verzerrt die Struktur und wird als "Mismatch" bezeichnet.
- Perfekter Match: Hybrid aus zwei komplementären Nukleinsäure-Oligomeren, bei dem kein Mismatch auftritt.
- ss: Single strand (Einzelstrang)
- ds: Double strand (Doppelstrang)
- redoxaktiv: Bezeichnet die Eigenschaft einer Einheit unter bestimmten äußeren Umständen an ein geeignetes Oxidationsmittel Elektronen abzugeben oder von einem geeigneten Reduktionsmittel Elektronen aufzunehmen.
- EDTA: Ethylendiamin-Tetraacetat (Natriumsalz)
- SNHS, sulfo-NHS: N-Hydroxysulfosuccinimid
- NHS: N-Hydroxysuccinimid
- EDC: (3-Dimethylaminopropyl)-carbodiimid
- HEPES: N-[2-Hydroxyethyl]piperazin-N'-[2-ethansulfonsäure]
- Tris: Tris-(hydroxymethyl)-aminomethan
- Linker, Spacer: Molekulare Verbindung zwischen zwei Molekülen bzw. zwischen einem Oberflächenatom, Oberflächenmolekül oder einer Oberflächenmolekülgruppe und einem anderen Molekül. In der Regel sind Linker als Alkyl-, Alkenyl-, Alkinyl-, Hetero-Alkyl-, Hetero-Alkenyl- oder Hetero-Alkinylkette käuflich zu erwerben, wobei die Kette an zwei Stellen mit (gleichen oder verschiedenen) reaktiven Gruppen derivatisiert ist. Diese Gruppen bilden in einfachen/bekannten chemischen Reaktionen mit dem entsprechenden Reaktionspartner eine kovalente chemische Bindung aus. Die reaktiven Gruppen können auch photoaktivierbar sein, d.h. die reaktiven Gruppen werden erst durch Licht bestimmter oder beliebiger Wellenlänge aktiviert. Auch unspezifische nt, i.e. nicht zu anderen Basen komplementäre nt, können als Linker/Spacer verwendet werden, insbesondere bei der Anbindung von Sonden-Oligos an eine Oberfläche.

Die vorliegende Erfindung stellt ein Verfahren zur Detektion von Nukleinsäureoligomer-Hybridisierungsereignissen bereit, das die Schritte Bereitstellen einer modifizierten Oberfläche, wobei die Modifikation in der Anbindung wenigstens einer Art von Sonden-Nukleinsäureoligomeren besteht, Bereitstellen wenigstens einer Art von Signal-Nukleinsäureoligomeren, wobei die Signal-Nukleinsäureoligomere mit zumindest einem Detektionslabel modifiziert sind und die Signal-Nukleinsäureoligomere einen zu den Sonden-Nukleinsäureoligomeren komplementären oder weitgehend komplementären Abschnitt besitzen, Bereitstellen einer Probe mit Target-Nukleinsäureoligomeren, Inkontaktbringen einer definierten Menge der Signal-Nukleinsäureoligomere mit der modifizierten Oberfläche, Inkontaktbringen der Probe und der darin enthaltenen Target-Nukleinsäureoligomere mit der modifizierten Oberfläche, Detektion der Signal-Nukleinsäureoligomere und Vergleich der bei der Detektion der Signal-Nukleinsäureoligomere erhaltenen Werte mit Referenzwerten umfasst. Erfindungsgemäß besitzen die Signal-Nukleinsäureoligomere eine größere Anzahl an Basen als die Sonden-Nukleinsäureoligomere und weisen zumindest einen Andockabschnitt auf, wobei der Andockabschnitt keine zu einem Abschnitt der Sonden-Nukleinsäureoligomere komplementäre oder weitgehend komplementäre Struktur aufweist und wobei die Target-Nukleinsäureoligomere einen zu dem Andockabschnitt komplementären oder weitgehend komplementären Abschnitt besitzen.

Unter einer "weitgehend komplementären Struktur" werden im Rahmen der vorliegenden Erfindung Sequenzabschnitte verstanden, bei denen maximal 20% der Basenpaare Mismatches ausbilden. Bevorzugt handelt es sich bei einer "weitgehend komplementären Struktur" im Rahmen der vorliegenden Erfindung um Sequenzabschnitte, bei denen maximal 15% der Basenpaare Mismatches ausbilden. Besonders bevorzugt handelt es sich bei einer "weitgehend komplementären Struktur" um Sequenzabschnitte, bei denen maximal 10% der Basenpaare Mismatches ausbilden und ganz besonders bevorzugt um Sequenzabschnitte, bei denen maximal 5% der Basenpaare Mismatches ausbilden.

Durch den erfindungsgemäßen Andockabschnitt kann die Geschwindigkeit, mit der die Assoziation der Target-Nukleinsäureoligomere an die Signal-Nukleinsäureoligomere erfolgt, um ein Vielfaches erhöht werden. In den aus dem Stand der Technik bekannten Verdrängungsassays zur Detektion von Nukleinsäureoligomer-Hybridisierungsereignissen liegen Sonden-Nukleinsäureoligomere und Signal-Nukleinsäureoligomere bei Zugabe der Target-Nukleinsäureoligomere bzw. Sonden-Nukleinsäureoligomere und Target-Nukleinsäureoligomere bei Zugabe der Signal-Nukleinsäureoligomere als hybridisierter Doppelstrang vor. Vor einer Bindung der zugegebenen Nukleinsäureoligomer-Komponente müssen entsprechend die Bindungen des hybridisierten Doppelstrangs gelöst werden.

Im Gegensatz dazu besitzen gemäß der vorliegenden Erfindung die beiden Nukleinsäureoligomer-Komponenten Sonden-Nukleinsäureoligomere und Signal-Nukleinsäureoligomere eine unterschiedliche Anzahl an Basen. Die Signal-Nukleinsäureoligomere weisen eine größere Zahl an Basen auf und stellen einen Andockabschnitt zur Verfügung, der in einem nicht-hybridisierten Zustand vorliegt, da er keine zu einem Abschnitt der Sonden-Nukleinsäureoligomere komplementäre oder weitgehend komplementäre Struktur aufweist.

Gleichzeitig weisen nun aber die Target-Nukleinsäureoligomere einen zu dem Andockabschnitt komplementären oder weitgehend komplementären Abschnitt auf. Bei Zugabe der Target-Nukleinsäureoligomere können diese direkt ohne vorherige Verdrängung einer hybridisierten Komponente an diesen Andockabschnitt binden. Im Zuge der nachfolgenden Hybridisierung mit den Signal-Nukleinsäureoligomeren muss zwar wie aus dem Stand der Technik bekannt, die hybridisierte Nukleinsäureoligomer-Komponente verdrängt werden. Allerdings erfolgt diese Verdrängung aufgrund der bereits erfolgten Hybridisierung mit dem Andockabschnitt mit einer deutlich höheren Geschwindigkeit.

Selbstverständlich können die Signal-Nukleinsäureoligomeren auch zwei oder sogar noch mehr Andockabschnitte aufweisen. Es soll darauf hingewiesen werden, dass der Andock-Bereich nicht unmittelbar an den zu den Sonden-Nukleinsäureoligomeren komplementären bzw. weitgehend komplementären Abschnitt ("Displace"-Bereich) der Signal-Nukleinsäureoligomere anschließen muss. Zwischen den beiden Bereichen kann ein Abschnitt mit beliebiger Basensequenz vorhanden sein, der beispielsweise auch ein Loop ausbilden kann.

Die Target-Nukleinsäureoligomere liegen in der Probe entweder als Einzelstrang (ss) oder als Doppelstrang (ds) vor. In einer bevorzugten Ausführungsform liegen die Target-Nukleinsäureoligomere zumindest teilweise als Einzelstrang vor. Dies kann z.B. dadurch erreicht werden, dass ds-Target-Nukleinsäureoligomere (thermisch oder durch sonstige, dem Fachmann bekannte Maßnahmen) dehybridisiert werden oder dass bei der Aufbereitung der Target-Nukleinsäureoligomere darauf geachtet wird, dass die Target-Nukleinsäureoligomere z.T. als Einzelstränge vorliegen. Dies wird beispielsweise durch eine asymmetrische PCR erreicht.

Wie bereits erläutert, besitzen die beiden Nukleinsäureoligomer-Komponenten Sonden-Nukleinsäureoligomere und Signal-Nukleinsäureoligomere eine unterschiedliche Anzahl an Basen, wobei der Andockabschnitt durch die Signal-Nukleinsäureoligomere, welche die größere Zahl an Basen aufweisen, zur Verfügung gestellt wird. Die Target-Nukleinsäureoligomere hybridisieren an die Signal-Nukleinsäureoligomere und verdrängen auf diese Weise die vorher gebundenen Sonden-Oligomere. Der hybridisierte Doppelstrang aus Target-Nukleinsäureoligomeren und Signal-Nukleinsäureoligomeren löst sich von der Oberfläche, an der nicht-hybridisierte Sonden-Oligomere zurückbleiben. Bei der Detektion der Signal-Oligomere stellt man bei Anweisenheit des gesuchten Targets eine Abnahme der Signalintensität mit der Zeit fest.

Gemäß bevorzugten Ausführungsformen der vorliegenden Erfindung unterscheidet sich die Zahl der Basen von Sonden-Nukleinsäureoligomeren und Signal-Nukleinsäureoligomeren um 6 bis 80, besonders bevorzugt um 9 bis 60 und insbesondere bevorzugt um 10 bis 40. Ein Längenunterschied in der angegebenen Zahl an Basen stellt einerseits einen genügend langen Andock-Abschnitt zur Verfügung und verlängert andererseits die verwendeten Nukleinsäureoligomere nicht unnötig.

Vorteilhafterweise wird nach dem Inkontaktbringen einer definierten Menge der Signal-Nukleinsäureoligomere mit der modifizierten Oberfläche, aber vor dem Inkontaktbringen der Probe und der darin enthaltenen Target-Nukleinsäureoligomere mit der modifizierten Oberfläche eine erste Detektion der Signal-Nukleinsäureoligomere zur Bestimmung von Referenzwerten durchgeführt. Im letzten Schritt des Verfahrens gemäß der vorliegenden Erfindung werden die bei der weiteren (zweiten) Detektion der Signal-Nukleinsäureoligomere erhaltenen Werte dann mit den bei der ersten Detektion der Signal-Nukleinsäureoligomere erhaltenen Referenzwerten verglichen.

Bevorzugt sind die Signal-Nukleinsäureoligomere mit mehreren Detektionslabel modifiziert, wodurch Signale mit höherer Intensität erhalten werden. Besonders bevorzugt wird als Detektionslabel ein Fluorophor verwendet, insbesondere ein Fluoreszenzfarbstoff wie insbesondere Texas Rot, ein Rhodamin-Farbstoff oder Fluorescein.

Gemäß einer weiteren bevorzugten Ausführungsform der vorliegenden Erfindung wird als Detektionslabel eine redoxaktive Substanz verwendet. Ebenfalls bevorzugt wird eine leitfähige Oberfläche als modifizierte Oberfläche verwendet, wodurch insbesondere die elektrochemische Detektion der Hybridisierungsereignisse erleichtert wird.

Bevorzugt erfolgt die Detektion der Signal-Nukleinsäureoligomere durch eine oberflächensensitive Detektionsmethode, da in diesem Fall ausschließlich die an die Oberfläche gebundenen Signal-Nukleinsäureoligomere detektiert werden. Besonders bevorzugt sind in diesem Zusammenhang spektroskopische, elektrochemische und elektrochemolumineszente Methoden. Als spektroskopisches Verfahren wird besonders eine Detektion der Fluoreszenz, insbesondere der Total Internal Reflection Fluorescence (TIRF) der Signal-Nukleinsäureoligomere bevorzugt.

Zur elektrochemischen Detektion werden bevorzugt Cyclovoltammetrie, Amperometrie, Chronocoulometrie, Impedanzmessung oder Scanning Electrochemical Microscopy (SECM) eingesetzt.

Die Signal-Nukleinsäureoligomere weisen bevorzugt 10 bis 200 Nukleinsäuren, insbesondere 20 bis 100 Nukleinsäuren, besonders bevorzugt 25 bis 70 Nukleinsäuren auf. Mit Hilfe von Signal-Nukleinsäureoligomeren dieser Länge können alle gewünschten Targets eindeutig identifiziert werden.

Ganz besonders bevorzugt werden Signal-Nukleinsäureoligomere und Sonden-Nukleinsäureoligomere verwendet, die in den miteinander hybridisierenden Bereich ihrer Sequenz zumindest ein nicht-komplementäres Basenpaar aufweisen. In diesem Fall weisen Signal-Nukleinsäureoligomere und Sonden-Nukleinsäureoligomere also eine weitgehend komplementäre Struktur im Sinne der vorliegenden Erfindung auf. Die Verdrängung der Signal-Nukleinsäureoligomere bzw. der Sonden-Nukleinsäureoligomere durch die Target-Nukleinsäureoligomere wird gemäß dieser Ausführungsform deutlich erleichtert, was wiederum zu einer erhöhten Reaktionsgeschwindigkeit führt.

Bevorzugt wird die Detektion der Signal-Nukleinsäureoligomere mehrfach wiederholt, wodurch die Bestimmung der Abnahme der Intensität mit der Zeit im Laufe der Verdrängung der Signal-Nukleinsäureoligomere bzw. der Sonden-Nukleinsäureoligomere durch die Target-Nukleinsäureoligomere ermöglicht wird. Die erste Detektion der Signal-Nukleinsäureoligomere erfolgt dabei bevorzugt in einer Puffer-Lösung vor dem Inkontaktbringen der Probe mit der modifizierten Oberfläche. Durch diese Messung wird ein Referenzwert erhalten, gegenüber dem die nachfolgenden Messungen nach Zugabe der Target-Nukleinsäureoligomere normiert werden können.

Die erste Detektion der Signal-Nukleinsäureoligomere nach dem Inkontaktbringen der Probe und der darin enthaltenen Target-Nukleinsäureoligomere mit der modifizierten Oberfläche erfolgt unabhängig von der Frage, ob eine Detektion in einer Puffer-Lösung durchgeführt wurde oder nicht, sofort nach dem Inkontaktbringen der Probe mit der modifizierten Oberfläche und wird anschließend über einen Zeitraum von zumindest 40 Min., bevorzugt zumindest 20 Min., besonders bevorzugt zumindest 10 Min. und ganz besonders bevorzugt zumindest 5 Min. mehrfach wiederholt. In den genannten Zeiträumen erfolgt in der Regel eine genügend große Intensitätsänderung bei der Detektion der Signal-Nukleinsäureoligomere, um eine genaue Aussage über das Vorhandensein eines bestimmten Target-Nukleinsäureoligomers treffen zu können.

Der Verdrängungsvorgang kann durch eine Temperaturerhöhung beschleunigt werden. Bevorzugt wird daher während der mehrfachen Wiederholung der Detektion der Signal-Nukleinsäureoligomere die Temperatur im Bereich der modifizierten Oberfläche ausgehend von Raumtemperatur verändert. Besonders gute Resultate werden erzielt, wenn die Temperatur im Bereich der modifizierten Oberfläche während der wiederholten Detektion der Signal-Nukleinsäureoligomere um 1°C bis 10°C pro Min., bevorzugt um 2 °C pro Min. über die Raumtemperatur erhöht wird.

In einer alternativen Form der Detektion wird während der mehrfachen Wiederholung der Detektion der Signal-Nukleinsäureoligomere die Temperatur im Bereich der modifizierten Oberfläche ausgehend von einer ersten Detektion der Signal-Nukleinsäureoligomere bei Raumtemperatur auf eine für die Detektion besonders geeignete Temperatur erhöht und die Detektion bei dieser Temperatur über einen gewissen Zeitraum von 2 Min. bis 60 Min. mehrfach wiederholt. Als besonders geeignete Temperatur gilt dabei eine Temperatur, die 5°C bis 40°C unter der Schmelztemperatur des Hybrids aus Sonden- und-Signal-Oligonukleotids liegt, insbesondere eine solche, die 5°C bis 30°C darunter liegt, besonders bevorzugt wird eine Temperatur, die 5°C bis 20°C unter der Schmelztemperatur des Hybrids liegt.

Sämtliche im Rahmen der vorliegenden Erfindung beschriebenen Verfahren können unter Verwendung von DNA-Chips durchgeführt werden. In diesem Fall weist die modifizierte Oberfläche zumindest 2 räumlich im wesentlichen abgetrennte Bereiche, bevorzugt zumindest 4 und insbesondere zumindest 12 räumlich im wesentlichen abgetrennte Bereiche auf.

Unter "räumlich im wesentlichen abgetrennten Bereichen" werden Bereiche der Oberfläche verstanden, die ganz überwiegend durch Anbindung einer bestimmten Art von Sonden-Nukleinsäureoligomeren modifiziert sind. Lediglich in Gebieten, in denen zwei solche räumlich im wesentlichen abgetrennte Bereiche aneinander grenzen, kann es zu einer Vermischung von verschiedenen Arten von Sonden-Nukleinsäureoligomeren kommen.

Ganz besonders bevorzugt weist die modifizierte Oberfläche zumindest 32, insbesondere zumindest 64, ganz besonders bevorzugt zumindest 96 räumlich im wesentlichen abgetrennte Bereiche auf.

Gemäß einer weiteren, besonders bevorzugten Ausführungsform ist in jeweils einem der räumlich im wesentlichen abgetrennten Bereiche der Oberfläche jeweils eine Art von Sonden-Nukleinsäureoligomeren an die Oberfläche gebunden, wobei sich die verschiedenen Arten von Sonden-Nukleinsäureoligomeren in zumindest einer Base voneinander unterscheiden. Dies erlaubt die parallele Detektion einer Vielzahl verschiedener Arten von Target-Nukleinsäureoligomeren.

Besonders bevorzugt werden die erfindungsgemäßen Verfahren unter Verwendung einer modifizierten Oberfläche durchgeführt, wobei die Modifikation in der Anbindung wenigstens einer Art von Sonden-Nukleinsäureoligomeren besteht, und wobei wenigstens eine Art von zumindest mit einem Detektionslabel modifizierten Signal-Nukleinsäureoligomeren an die Sonden-Nukleinsäureoligomere hybridisiert vorliegt. Da in diesem Fall die Signal-Nukleinsäureoligomere bereits an die Sonden-Nukleinsäureoligomere hybridisiert vorliegen, kann auf das nachfolgende Zugeben der Signal-Nukleinsäureoligomere verzichtet werden. Für den Anwender des Verfahrens vereinfacht sich der Vorgang dadurch weiter.

Die vorliegende Erfindung umfasst auch eine modifizierte Oberfläche, wobei die Modifikation in der Anbindung wenigstens einer Art von Sonden-Nukleinsäureoligomeren besteht, und wobei wenigstens eine Art von zumindest mit einem Detektionslabel modifizierten Signal-Nukleinsäureoligomeren an die Sonden-Nukleinsäureoligomere hybridisiert vorliegt, wobei die Signal-Nukleinsäureoligomere eine größere Zahl an Basen besitzen als die Sonden-Nukleinsäureoligomere und die Signal-Nukleinsäureoligomere zumindest einen Andockabschnitt aufweisen, wobei der Andockabschnitt keine zu einem Abschnitt der Sonden-Nukleinsäureoligomere komplementäre oder weitgehend komplementäre Struktur aufweist.

Bevorzugt unterscheidet sich die Zahl der Basen von Sonden-Nukleinsäureoligomeren und Signal-Nukleinsäureoligomeren um 6 bis 80, besonders bevorzugt um 9 bis 60 und ganz besonders bevorzugt um 10 bis 40. Ebenfalls bevorzugt sind Ausführungsformen, bei denen die Signal-Nukleinsäureoligomere mit mehreren Detektionslabel modifiziert sind.

Besonders bevorzugt werden modifizierte Oberflächen, bei denen die an die Oberfläche gebundenen Signal-Nukleinsäureoligomere ein Fluorophor als Detektionslabel tragen, insbesondere einen Fluoreszenzfarbstoff wie insbesondere Texas Rot, einen Rhodamin-Farbstoff oder Fluorescein. Ebenfalls bevorzugt kann als Detektionslabel aber auch eine redoxaktive Substanz verwendet werden. Da in diesem Fall eine elektrochemische Detektion durchgeführt wird, handelt es sich bei der modifizierten Oberfläche bevorzugt um eine leitfähige Oberfläche.

Die Signal-Nukleinsäureoligomere weisen bevorzugt 10 bis 200 Nukleinsäuren, insbesondere 20 bis 100 Nukleinsäuren, besonders bevorzugt 25 bis 70 Nukleinsäuren auf.

Bevorzugt weisen Signal-Nukleinsäureoligomere und Sonden-Nukleinsäureoligomere in den miteinander hybridisierenden Bereich ihrer Sequenz zumindest ein nicht-komplementäres Basenpaar auf.

Die modifizierte Oberfläche besitzt bevorzugt zumindest 2 räumlich im wesentlichen abgetrennte Bereiche, besonders bevorzugt zumindest 4 und insbesondere zumindest 12 solcher räumlich im wesentlichen abgetrennte Bereiche. Ganz besonders bevorzugt weist die modifizierte Oberfläche zumindest 32, insbesondere zumindest 64, ganz besonders bevorzugt zumindest 96 räumlich im wesentlichen abgetrennte Bereiche aufweist.

Gemäß einer bevorzugten Ausführungsform ist in jeweils einem der räumlich im wesentlichen abgetrennten Bereiche der Oberfläche jeweils eine Art von Sonden-Nukleinsäureoligomeren an die Oberfläche gebunden, wobei sich die verschiedenen Arten von Sonden-Nukleinsäureoligomeren in zumindest einer Base voneinander unterscheiden.

Mit den verschiedenen bevorzugten Ausführungsformen der erfindungsgemäßen modifizierten Oberfläche sind grundsätzlich die selben Vorteile verbunden wie mit den entsprechenden bevorzugten Ausführungsformen der erfindungsgemäßen Verfahren. In diesem Zusammenhang wird auf die obigen Ausführungen im Zusammenhang mit den erfindungsgemäßen Verfahren verwiesen.

Daneben betrifft die vorliegende Erfindung auch einen Kit zur Durchführung eines der oben beschriebenen Verfahren zur Detektion von Nukleinsäureoligomer-Hybridisierungsereignissen. Der Kit umfasst eine modifizierte Oberfläche, wobei die Modifikation in der Anbindung wenigstens einer Art von Sonden-Nukleinsäureoligomeren besteht, und eine effektive Menge an Signal-Nukleinsäureoligomeren mit den oben näher erläuterten Eigenschaften.

Daneben betrifft die vorliegende Erfindung auch einen Kit zur Durchführung eines der oben beschriebenen Verfahren zur Detektion von Nukleinsäureoligomer-Hybridisierungsereignissen. Der Kit umfasst eine modifizierte Oberfläche, wobei die Modifikation in der Anbindung wenigstens einer Art von Sonden-Nukleinsäureoligomeren besteht, und wobei wenigstens eine Art von zumindest mit einem Detektionslabel modifizierten Signal-Nukleinsäureoligomeren an die Sonden-Nukleinsäureoligomere hybridisiert vorliegt, wobei die Signal-Nukleinsäureoligomere eine größere Zahl an Basen besitzen als die Sonden-Nukleinsäureoligomere und die Signal-Nukleinsäureoligomere zumindest einen Andockabschnitt aufweisen, wobei der Andockabschnitt keine zu einem Abschnitt der Sonden-Nukleinsäureoligomere komplementäre oder weitgehend komplementäre Struktur aufweist.

Gemäß einer bevorzugten Ausführungsform sind die Referenzwerte von dem Kit bereits umfasst, sodass die Signal-Nukleinsäureoligomere vom Endverbraucher nur einmal detektiert werden müssen. Die bei dieser Detektion erhaltenen Werte brauchen dann nur mit den bereits vorhandenen Referenzwerten verglichen zu werden.

### Die Oberfläche

Mit dem Begriff "Oberfläche" wird jedes Trägermaterial bezeichnet, das geeignet ist direkt oder nach entsprechender chemischer Modifizierung derivatisierte oder nichtderivatisierte Sonden-Nukleinsäureoligomere kovalent oder über andere spezifische Wechselwirkungen zu binden. Der feste Träger kann aus leitfähigem oder nicht leitfähigem Material bestehen.

### (i) leitfähige Oberflächen

Unter dem Begriff "leitfähige Oberfläche" wird jeder Träger mit einer elektrisch leitfähigen Oberfläche beliebiger Dicke verstanden, insbesondere Oberflächen aus Platin, Palladium, Gold, Cadmium, Quecksilber, Nickel, Zink, Kohlenstoff, Silber, Kupfer, Eisen, Blei, Aluminium und Mangan.

Daneben können auch beliebige dotierte oder nicht dotierte Halbleiteroberflächen beliebiger Dicke verwendet werden. Sämtliche Halbleiter können als Reinsubstanzen oder als Gemische Verwendung finden. Als nicht einschränkend gemeinte Beispiele seien an dieser Stelle Kohlenstoff, Silizium, Germanium, α-Zinn, Cu(I)- und Ag(I)-Halogenide beliebiger Kristallstruktur genannt. Geeignet sind ebenfalls sämtliche binären Verbindungen beliebiger Zusammensetzung und beliebiger Struktur aus den Elementen der Gruppen 14 und 16, den Elementen der Gruppen 13 und 15, sowie den Elementen der Gruppen 15 und 16. Daneben können ternäre Verbindungen beliebiger Zusammensetzung und beliebiger Struktur aus den Elementen der Gruppen 11, 13 und 16 oder den Elementen der Gruppen 12, 13 und 16 verwendet werden. Die Bezeichnungen der Gruppen des Periodensystems der Elemente beziehen sich auf die IUPAC-Empfehlung von 1985.

### (ii) nichtleitende Oberflächen

Bei den nichtleitenden Oberflächen wird als Material Glas, modifiziertes Glas oder Silizium bevorzugt. Die Modifizierung kann z.B. durch Silanisierung erfolgen und führt in allen Fällen zu funktionellen Gruppen, die geeignet sind, in Kopplungsreaktionen entsprechend funktionalisierte Sonden-Nukleinsäureoligomere zu binden. Diese Modifizierung schließt Schichtaufbauten auf der Oberfläche unter Verwendung von Polymeren wie z.B. Dextranpolymeren, die eine Variation der Schichtdicke und Oberflächenbeschaffenheit erlauben, mit ein. Weitere Derivatisierungsmöglichkeiten zur letztendlichen Anbindung der Sonden-Nukleinsäureoligomeren bestehen z.B. im Aufbringen einer dünnen (ca. 10 - 200 nm) Metallisierungsschicht, insbesondere einer Gold-Metallisierungsschicht, die zusätzlich mit (Thiol-funktionalisierten) Polymeren, insbesondere Dextranen, belegt sein kann. Daneben kann das Glas nach der Silanisierung auch mit Biotin funktionalisiert werden (z.B. aminofunktionalisierte Glasoberfläche nach der Silanisierung und Kopplung der Carbonsäure Biotin über einen Biotinaktivester wie Biotin-N-succinimidylester) oder alternativ mit an Dextran-Lysin oder Dextran immobilisierten Biotin überzogen werden. Die so erzeugten biotinylierten Glasoberflächen werden anschließend mit Avidin oder Streptavidin behandelt und können dann zur Anbindung von biotinylierten Sonden-Nukleinsäureoligomeren verwendet werden.

### Bindung von Nukleinsäureoligomeren an die Oberfläche

Verfahren zur Immobilisierung von Nukleinsäureoligomeren an einer Oberfläche sind dem Fachmann bekannt. Die Sonden-Nukleinsäureoligomere können z.B. kovalent über Hydroxyl-, Epoxid-, Amino- oder Carboxygruppen des Trägermaterials mit natürlicherweise am Nukleinsäureoligomer vorhandenen oder durch Derivatisierung am Sonden-Nukleinsäureoligomer angebrachten Thiol-, Hydroxy-, Amino- oder Carboxylgruppen an die Oberfläche gebunden werden. Das Sonden-Nukleinsäureoligomer kann direkt oder über einen Linker/Spacer an die Oberflächenatome oder -molekülen einer Oberfläche gebunden werden. Daneben kann das Sonden-Nukleinsäureoligomer durch die bei Immunoassays üblichen Methoden verankert werden wie z.B. durch Verwendung von biotinylierten Sonden-Nukleinsäureoligomeren zur nicht-kovalenten Immobilisierung an Avidin oder Streptavidin-modifizierten Oberflächen. Die chemische Modifikation der Sonden-Nukleinsäureoligomere mit einer Oberflächen-Ankergruppe kann bereits im Verlauf der automatisierten Festphasensynthese oder aber in gesonderten Reaktionsschritten eingeführt werden. Dabei wird auch das Nukleinsäureoligomer direkt oder über einen Linker/Spacer mit den Oberflächenatomen oder -molekülen einer Oberfläche der oben beschriebenen Art verknüpft. Diese Bindung kann auf verschiedene dem Fachmann bekannte Arten durchgeführt werden. In diesem Zusammenhang wird auf die WO 00/42217 A1 verwiesen.

### Sonden-, Target- und Signal-Nukleinsäureoligomere

Die Sonden-Nukleinsäureoligomere der vorliegenden Erfindung bestehen aus Nukleotiden in einer bestimmten Nukleotidabfolge (Sequenz) und liegen an einer Oberfläche immobilisiert vor. Als Target-Nukleinsäureoligomere werden Moleküle bezeichnet, die spezifisch mit den Sonden-Nukleinsäureoligomeren oder mit den Signal-Nukleinsäureoligomeren unter Ausbildung eines Doppelstrang-Hybrids wechselwirken. Target-Nukleinsäureoligomere im Sinne der vorliegenden Erfindung sind also Nukleinsäureoligomere, die als Komplexbindungspartner des komplementären Sonden-Nukleinsäureoligomers bzw. Signal-Nukleinsäureoligomers fungieren. Die Target-Nukleinsäureoligomere, deren Vorhandensein anhand der vorliegenden Erfindung detektiert werden soll, weisen zumindest einen Sequenzbereich auf, dessen Sequenz komplementär oder zumindest weitgehend komplementär zu einem Abschnitt der Sonden-Nukleinsäureoligomere bzw. der Signal-Nukleinsäureoligomere ist.

Die Target-Nukleinsäureoligomere liegen in der Probe entweder als Einzelstrang (ss) oder als Doppelstrang (ds) vor. In einer bevorzugten Ausführungsform liegen die Target-Nukleinsäureoligomere zumindest teilweise als Einzelstrang vor. Dies kann z.B. dadurch erreicht werden, dass ds-Target-Nukleinsäureoligomere (thermisch oder durch sonstige, dem Fachmann bekannte Maßnahmen) dehybridisiert werden oder dass bei der Aufbereitung der Target-Nukleinsäureoligomere darauf geachtet wird, dass die Target-Nukleinsäureoligomere z.T. als Einzelstränge vorliegen. Dies wird beispielsweise durch eine asymmetrische PCR erreicht.

Als Nukleinsäureoligomer oder ns-Oligomer wird im Rahmen der vorliegenden Erfindung eine Verbindung aus wenigstens zwei kovalent verbundenen Nukleotiden oder aus wenigstens zwei kovalent verbundenen Pyrimidin- (z.B. Cytosin, Thymin oder Uracil) oder Purin-Basen (z.B. Adenin oder Guanin), bevorzugt ein DNA-, RNA-oder PNA-Fragment, verwendet. Der Begriff Nukleinsäure bezieht sich auf ein beliebiges "Rückgrat" der kovalent verbundenen Pyrimidin- oder Purin-Basen, wie z.B. auf das Zucker-Phosphat Rückgrat der DNA, cDNA oder RNA, auf ein Peptid-Rückgrat der PNA oder auf analoge Rückgrat-Strukturen, wie z.B. ein Thio-Phosphat-, ein Dithio-Phosphat- oder ein Phosphoramid-Rückgrat. Wesentliches Merkmal einer Nukleinsäure im Sinne der vorliegenden Erfindung ist die sequenzspezifische Bindung natürlich vorkommender DNA, oder RNA bzw. daraus abgeleitete (transkribierte oder amplifizierte) Strukturen wie cDNA oder amplifizierte cDNA oder amplifizierte RNA (aRNA).

### Detektions-Label / Markierung (Markermolekül)

Die Signal-Nukleinsäureoligomere sind durch Derivatisierung mit einem oder mehreren detektierbaren Label ausgestattet. Dieses Label ermöglicht die Detektion der Komplexierungsereignisse zwischen dem Signal-Nukleinsäureoligomeren und den oberflächengebundenen Sonden-Nukleinsäureoligomeren. Das Label kann direkt oder wie im Falle enzym-katalysierter Reaktionen indirekt ein Detektionssignal liefern. Bevorzugte Detektionslabel (Markermoleküle) sind Fluorophore und redoxaktive Substanzen.

Bei den Fluorophoren können kommerziell erhältliche Fluoreszenzfarbstoffe wie z.B. Texas Rot, Rhodamin-Farbstoffe, Fluorescein etc. verwendet werden (vgl. Molecular Probes Katalog). Im Falle der Detektion durch elektrochemische Methoden werden Redoxmoleküle als Label eingesetzt.

Als Redoxlabel können Übergangsmetall-Komplexe, insbesondere solche des Kupfers, Eisens, Rutheniums, Osmiums oder Titans mit Liganden wie Pyridin, 4,7-Dimethylphenanthrolin, 9,10-Phenanthrenquinondiimin, Porphyrine und substituierte Porphyrin-Derivate verwendet werden. Daneben ist der Einsatz von Riboflavin, von Chinonen wie Pyrrollochinolinochinon, Ubichinon, Anthrachinon, Naphtochinon oder Menachinon bzw. Derivaten davon, von Metallocenen und Metallocenderivaten wie Ferrocenen und Ferrocenderivaten, Cobaltocenen und Cobaltocenderivaten, von Porphyrinen, Methylenblau, Daunomycin, Dopamin-Derivaten, Hydrochinon-Derivaten (para- oder ortho-Dihydroxy-Benzol-Derivaten, para- oder ortho-Dihydroxy-Anthrachinon-Derivaten, para- oder ortho-Dihydroxy-Naphtochinon-Derivaten) und ähnlichen Verbindungen möglich.

In den erfindungsgemäßen Verfahren können auch indirekte Label verwendet werden. Unter dem Begriff "indirekte Label" werden solche verstanden, bei denen die eigentlich detektierbare Form des Labels erst über eine enzymkatalysierte Reaktion ensteht. Die detektierbare Form des Labels kann dann an der Oberfläche detektiert werden. Beispiele für solche indirekten Label sind dem Fachmann aus der Literatur bekannt, exemplarisch sei hier alkalische Phosphatase (AP) in Verbindung mit dem Substrat p-Aminophenylphosphat genannt. Liegt AP als indirekter Marker an das Signal-Nukleinsäureoligomer gebunden vor, so kann eine elektrochemische Detektion des Signal-Nukleinsäureoligomers dadurch erfolgen, dass zum Zeitpunkt der Detektion p-Aminophenylphosphat zugegeben wird. Das elektrochemisch inaktive p-Aminophenylphosphat dient als Substrat des Enzyms AP und wird in p-Aminophenol umgewandelt. p-Aminophenol kann nun, nach Diffusion zu einer leitfähigen Oberfläche, elektrochemisch detektiert werden, da diese Form des Substrats (also nach Umsetzung am AP) elektrochemisch aktiv ist. Alternativ kann AP auch zur chromogenen Detektion verwendet werden (z.B. mit 5-Brom-4-chlor-3-indoxylphosphat in Verbindung mit Nitroblau-Tetrazoliumchlorid).

### Oberflächensensitive Detektionsmethoden

Oberflächensensitive Detektionsmethoden erlauben die Unterscheidung zwischen an eine Oberfläche assoziierten und im Überstand gelösten Markermolekülen. Als Detektionsmethode eigenen sich elektrochemische, spektroskopische und elektrochemolumineszente Verfahren. Für die hier geschilderten Ausführungsformen sind oberflächensensitive Detektionsmethoden nicht zwingend notwendig, da in den beschriebenen Ausführungsformen die Konzentration der Signal-Oligonukleotide in der Volumenphase i.d.R. vergleichsweise gering ist: Es wird mit an der Oberfläche gebundenen (markierten) Signal-Oligonukleotiden begonnen, die im Laufe der Detektion sukzessive von der Oberfläche abgelöst und in die Volumenphase entlassen werden. Die Konzentration der Signal-Oligonukleotide in der Volumenphase ist deshalb (insbesondere zu Beginn der Detektion) gering und führt i.d.R. zu keiner detektierbaren Verfälschung des Messignals durch Beiträge der Volumenphase. Die Verwendung oberflächensensitiver Detektionsverfahren kann die Genauigkeit des Messergebnisses aber erhöhen.

### (i) Obenflächensensitive elektrochemische Detektion

Bei elektrochemischen Methoden kann anhand der Kinetik der elektrochemischen Prozesse prinzipiell zwischen an eine Oberfläche adsorbierten und im Überstand gelösten redoxaktiven Detektionslabel unterschieden werden. Oberflächenadsorbierte Detektionslabel werden im allgemeinen schneller elektrochemisch umgesetzt (z.B. oxidiert oder reduziert) als redoxaktive Detektionslabel aus der Volumenphase, da letztere vor der elektrochemischen Umsetzung erst zur (Elektroden-) Oberfläche diffundieren müssen. Als Beispiele für elektrochemische oberflächensensitive Methoden seien die Cyclovaltammetrie, die Amperometrie und die Chronocoulometrie genannt.

Die Methode der Chronocoulometrie z.B. erlaubt es, oberflächennahe redoxaktive Komponenten von (identischen) redoxaktiven Komponenten in der Volumenphase zu unterscheiden und ist z.B. in Steel, A.B., Herne, T.M. und Tarlov M.J.: Electrochemical Quantitation of DNA Immobilized on Gold, Analytical Chemistry, 1998, Vol. 70, 4670 - 4677 und darin zitierten Literaturstellen beschrieben. Der Einsatz der Chronocoulometrie in einem Verdrängungsassay zur Detektion von Nukleinsäureoligomerhybridisierungsereignissen ist detailliert in der WO 03/018834 A2 beschrieben, auf die hiermit in diesem Zusammenhang Bezug genommen wird.

### (ii) Oberflächensensitive Fluoreszenz-Detektion

Als optische Messmethode zur Detektion von fluoreszenzmarkierten Signal-Nukleinsäureoligomeren kann die Total Internal Reflection Fluorescence (TIRF, vgl. Sutherland und Dahne, 1987, J. Immunol. Meth., 74, 253 - 265) dienen. Dabei können Fluoreszenzmoleküle, die sich in der Nähe der Grenzfläche zwischen einem festen Wellenleitermedium, typischerweise Glas, und einem flüssigen Medium befinden bzw. auf der der Flüssigkeit zugewandten Oberfläche des Wellenleitermediums immobilisiert sind, durch das evaneszente Feld, das aus dem Wellenleiter herausragt, angeregt werden und detektierbares Fluoreszenzlicht emittieren. Verdrängte bzw. im Überstand gelöste fluoreszenzgelabelte Komplexbildner werden vom evaneszenten Feld nicht erfasst (bzw. nur insofern als sie sich im Bereich der Eindringtiefe des evaneszenten Feldes befinden) und liefern somit (nahezu) keinen Beitrag zum gemessenen Signal. Die Eindringtiefe des evaneszenten Feldes beträgt typischerweise 100 bis 200 nm, kann aber durch eine dünne Metallisierungsschicht (ca. 10 bis 200 nm), insbesondere eine Goldmetallisierungsschicht, auf mehrere 100 nm erhöht werden. In einer bevorzugten Ausführungsform der Fluoreszenzdetektion der verdrängten, Fluorophor-markierten Signal-Nukleinsäureoligomere wird die Schichtdicke der Sonden-modifizierten Trägeroberfläche an die Eindringtiefe des evaneszenten Feldes angepasst z.B.
- durch entsprechend lange Sonden-Nukleinsäureoligomere,
- durch Immobilisierung der Sonden-Nukleinsäureoligomere über entsprechend lange Linker zwischen Oberfläche und Sonden-Oligonukleotid,
- durch Kopplung der Carbonsäure Biotin (über einen Biotinaktivester wie Biotin-N-succinimidylester) an aminoderivatisierte Oberflächen und Kopplung von Avidin oder Streptavidin an die so erzeugten biotinylierten Oberflächen mit anschließender Anbindung von biotinylierten Sonden-Nukleinsäureoligomeren oder
- durch Immobilisierung einer entsprechend dicken Schicht an funktionalisiertem Polymer und Anbindung des Sonden-Nukleinsäureoligomeren an das Polymer, z.B. (a) durch Aufbringen einer dünnen (ca. 10 - 200 nm) Metallisierungsschicht, insbesondere einer Gold-Metallisierungsschicht, die mit einem (Thiol-funktionalisierten) Polymer, insbesondere Dextranen oder Polylysin, belegt sein kann, welches wiederum zur Anbindung der Sonden-Nukleinsäureoligomere verwendet wird oder (b) durch Aufbringen einer Polymerschicht aus Polylysin-, Dextran-Lysin- oder von Dextran-immobilisiertem Biotin und Kopplung von Avidin oder Streptavidin an die so erzeugten biotinylierten Oberflächen mit anschließender Anbindung von biotinylierten Sonden-Nukleinsäureoligomeren.

### Kurze Beschreibung der Zeichnungen

Die Erfindung soll nachfolgend anhand von Ausführungsbeispielen im Zusammenhang mit den Zeichnungen näher erläutert werden. Es zeigen
- Fig. 1: in schematischer Darstellung einen Chip zur Detektion von Nukleinsäureoligomer-Hybridisierungsereignissen mittels Verdrängungsassay;
- Fig. 2: eine schematische Darstellung der einzelnen Schritte des erfindungsgemäßen Verfahrens;
- Fig. 3A: ein Target-Nukleinsäureoligomer (Sequenz 5' nach 3');
- Fig. 3B: Varianten der erfindungsgemäßen modifizierten Oberfläche (i), (ii), (iv) und (v) mit einer on-chip Referenz (iii);

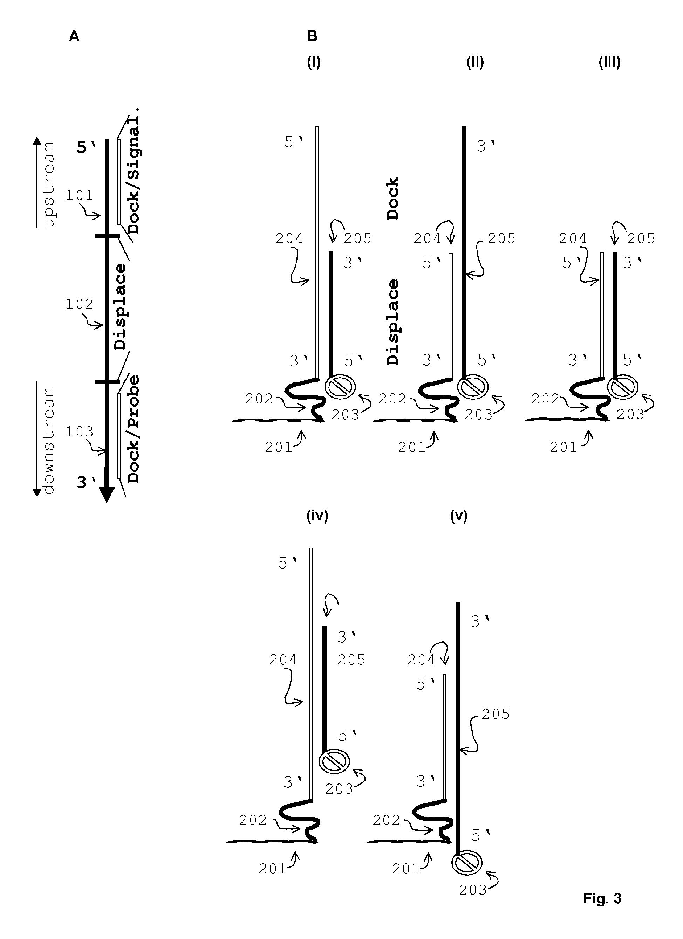

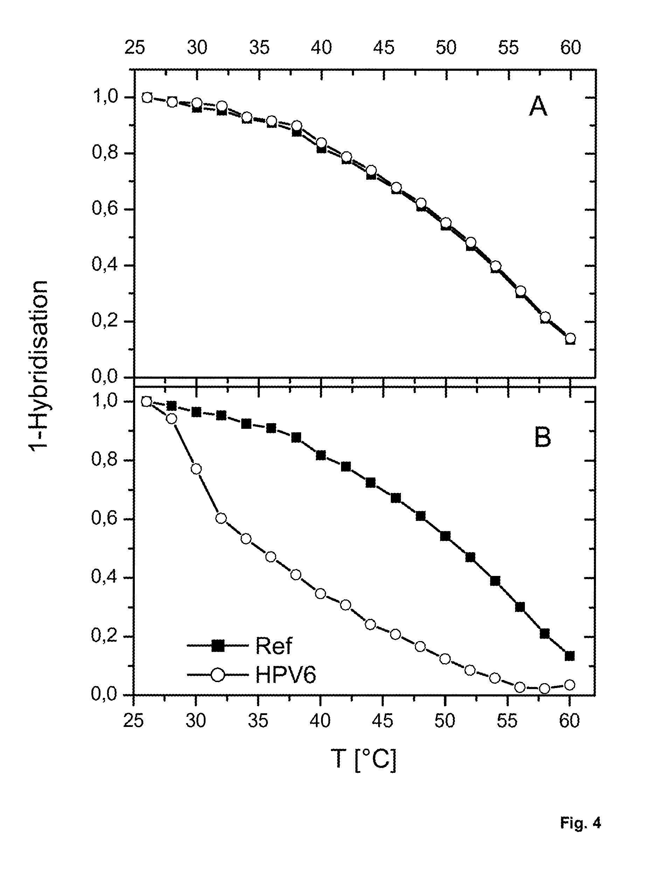

- Fig. 4A: Messergebnisse eines Verdrängungsassays für ein HPV 6 Target: Verlauf einer Detektionssequenz für eine HPV-6 Teststelle (-O-), deren Sonden-und Signal-Nukleinsäureoligomere dem Schema in Fig. 3B (ii) (Signal-Nukleinsäureoligomer mit Andockstelle) entspricht im Vergleich zu einer Referenzteststelle (- ■ -) deren Sonden- und Signal-Nukleinsäureoligomere dem Schema in Fig. 3 B (iii) entspricht. Im Analyt ist kein HPV-6 Target enthalten. Aufgetragen ist das elektrochemisch detektierte Signal des Ferrocen-markierten Signal-Nukleinsäureoligomers bei Anlegen eines Temperaturgradienten von 2°C/min an die Teststelle bezogen auf das bei 26°C erzielte Signal.
- Fig. 4B: Messergebnisse eines Verdrängungsassay für ein HPV_6 Target: Messung wie für Fig. 4A beschrieben, aber mit 10 nM Target in der Analytlösung (alle sonstigen Parameter sind identisch)
- Fig. 5: Messergebnisse eines Verdrängungsassays für ein HPV_6 Target: Messung wie für Fig. 4B beschrieben. Statt eines Temperaturgradienten wurde nach der Referenzmessung bei 26°C ein Temperatursprung auf 38°C durchgeführt und die weiteren Messpunkte bei dieser Temperatur in Abhängigkeit von der Zeit erhalten. Alle Werte sind bezogen auf das bei 26°C erzielte Signal.

### Bezugszeichenliste

I: Array von Test-Sites (Aufsicht)
II: Teststelle im Querschnitt
III: Immobilisierung von Sonden-Nukleinsäureoligomeren auf einer Teststelle (z.B. über thiolfunktionalisierte Sonden-Nukleinsäureoligomere, die auf Gold-Test-Sites gebunden werden, Ausschnitt aus einer Test-Site)
IV : Immobilisierung von Signal-Nukleinsäureoligomer durch Hybridisierung an Sonden-Nukleinsäureoligomere (hier: Signal-Nukleinsäureoligomere besitzen eine längere Sequenz als die Sonden-Nukleinsäureoligomere; der die Sonden-Nukleinsäureoligomere überragende Bereich wird als Andock-Bereich für das Target bezeichnet)
1: Teststelle zur Anbindung von Sonden
2: Test-Site-Abgrenzung (nicht zur Anbindung von Sonden geeignet)
3: Sonden-Nukleinsäureoligomer
4: Signal-Nukleinsäureoligomer
5: Marker (hier Ferrocen), der kovalent an das Signal-Nukleinsäureoligomer gebunden ist.
6: Hybrid aus Signal-Nukleinsäureoligomer und Target-Nukleinsäureoligomer
101: Sequenzbereich "upstream" von 102, kann zur Hybridisierung an den Andock-Abschnitt des Sonden- bzw. Signal-Nukleinsäureoligiomers benutzt werden.
102: Sequenz, die bevorzugt zur Diskriminierung von anderen Targets dient.
103: Sequenzbereich "downstream" von 102, kann zur Hybridisierung an den Andock-Abschnitt des Sonden bzw. Signal-Nukleinsäureoligomers benutzt werden.
201: Verankerung an der Oberfläche (z.B. mehrere Thiolfunktionen)
202: Linker/Spacer (z.B. mehrere unspezifische Basen) hybridisieren nicht mit Sequenzabschnitten des Signal- und auch nicht mit denen des Target-Nukleinsäureoligomers
203: Marker / Label (z.B. Ferrocen, kovalent an 205 gebunden).
204: Sonden-Nukeinsäureoligomer
205: Signal-Nukleinsäureoligomer

### Wege zur Ausführung der Erfindung

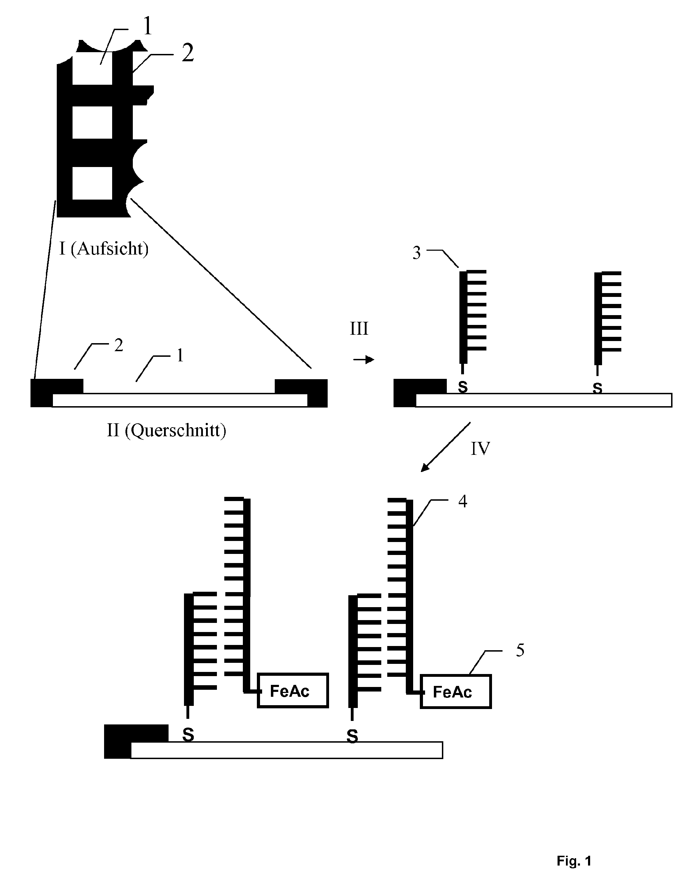

Um die Vorteile der DNA-Chip-Technologie auf die Detektion von Nukleinsäureoligomer-Hybriden durch den Verdrängungsassay anzuwenden, werden verschiedene modifizierte Sonden-Nukleinsäureoligomere unterschiedlicher Sequenz mit den oben beschriebenen Immobilisierungstechniken an einen Träger gebunden (vgl. Fig. 1, III). Mit der Anordnung der Sonden-Nukleinsäureoligomere bekannter Sequenz an definierten Positionen der Oberfläche, einem DNA-Array, soll das Hybridisierungsereignis eines beliebigen Target-Nukleinsäureoligomers detektierbar sein, um Targets wie z.B. Viren-RNA oder -DNA oder auch Mutationen im Target-Nukleinsäureoligomer aufzuspüren und sequenzspezifisch nachzuweisen. Dazu werden auf einer Oberfläche die Oberflächenatome oder -moleküle eines definierten Bereichs (einer Test-Site) mit DNA-/RNA-/PNA-Nukleinsäureoligomeren bekannter, aber beliebiger Sequenz, wie oben beschrieben, verknüpft. Der DNA-Chip kann auch mit einem einzigen Sonden-Oligonukleotid derivatisiert werden. Als Sonden-Nukleinsäureoligomere werden Nukleinsäureoligomere (z.B. DNA-, RNA-oder PNA-Fragmente) der Basenlänge 3 bis 200 oder 3 bis 100 oder 3 bis 70, bevorzugt der Länge 10 bis 70 oder 10 bis 25 verwendet.

Die so bereitgestellte Oberfläche mit immobilisierten Sonden-Oligonukleotiden wird mit einer Lösung einer bestimmten Menge von Signal-Nukleinsäureoligomeren, z.B. mit Redox-Label markierten Nukleinsäureoligomeren inkubiert (Fig. 1, IV). Dabei kommt es zur Ausbildung von Hybriden aus Sonden-Nukleinsäureoligomeren und Signal-Nukleinsäureoligomeren im Bereich komplementärer Sequenzen. Das Signal-Nukleinsäure-Oligomer besitzt eine längere Sequenz als das Sonden-Nukleinsäureoligomer und weist daher über den hybridisierten Bereich hinaus Sequenzabschnitte auf, die -bei geeigneter Basenabfolge- zur weiteren Hybridisierung z.B. mit einem Target-Nukleinsäureoligomer dienen können (Andock-Abschnitt). Nicht gebundene Signal-Nukleinsäureoligomere können gegebenenfalls durch Spülen mit geeigneter Pufferlösung von der derivatisierten Oberfläche entfernt werden.

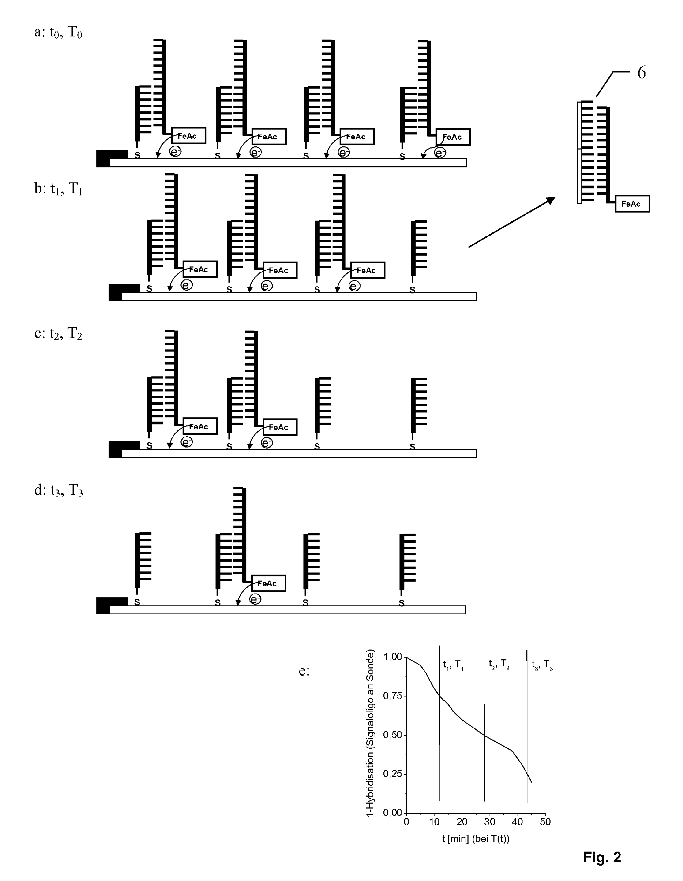

In Figur 2 sind die verschiedenen Schritte eines erfindungsgemäßen Verfahrens dargestellt. Zu einem Anfangszeitpunkt t₀ (bei der Anfangstemperatur T₀, vor Zugabe des Analyten) liegen die Signal-Oligonukleotide im Hybrid mit den an der Oberfläche immobilisierten Sonden-Oligonukleotide vor (Fig. 2a). Die Signal-Oligonukleotide weisen eine längere Sequenz als die Sonden-Oligonukleotide auf und sind kovalent mit einem Ferrocen (FcAc) derivatisiert (vgl. Fig. 1). Die Sonden-Oligonukleotide sind an eine leitfähige Oberfläche immobilisiert und mit Signal-Nukleinsäureoligomer hybridisiert. Durch Anlegen eines geeigneten Potentials kann Ferrocen in einer elektrochemischen Messung oxidiert werden und dient als Maß für das Vorliegen von (an Sonden-Oligonukleotide hybridisierten) Signal-Oligonukleotiden. Die Messung bei t₀ und T₀ (z.B. eine erste cyclovoltammetrische oder chronocoulometrische Messung) kann als Referenzmessung dienen und den oberflächenimmobilisierten Anteil der Signal-Nukleinsäureoligomere bestimmen.

Im nächsten Schritt wird die (möglichst konzentrierte) Untersuchungslösung mit Target-Oligonukleotid(en) zur Oberfläche mit immobilisierten Sonden-Oligonukleotiden und hybridisierten Signal-Nukleinsäureoligomeren gegeben. Enthält die Lösung Target-Nukleinsäureoligomer-Stränge, die zu den indirekt (durch Hybridisierung) an die Oberfläche gebundenen Signal-Nukleinsäureoligomeren komplementär oder zumindest in weiten Bereichen (bzw. weiteren Bereichen als das Sonden-Oligonukleotid) komplementär sind, kommt es zu einer Hybridisierung mit dem Signal-Nukleinsäureoligomer.

Dabei hybridisiert das Target zunächst an den Andock-Abschnitt der Signal-Oligonukleotide. Das Hybrid 6 aus Target- und Signal-Nukleinsäureoligomer geht in die Volumenphase über. Anschließend erfolgt dann die vollständige Hybridisierung von Target-Oligonukleotiden und Signal-Oligonukleotiden und Verdrängung der ursprünglich an die Sonden-Oligonukleotide hybridisierten Signal-Oligonukleotide. Zu einem späteren Zeitpunkt t₁ (bei der Temperatur T₁, die von T₀ abweichen kann), ist also ein Teil der Signal-Oligonukleotide der Teststelle verdrängt, sofern das passende Target im Analyten vorhanden war (Fig. 2b). Dies geschieht durch Hybridisierung des Targets mit dem Signal-Oligonukleotid.

Zu einem Zeitpunkt t₂ (bei der Temperatur T₂, die von T₁ abweichen kann), ist ein weiterer Teil der Signal-Oligonukleotide der Teststelle verdrängt (Fig. 2c). Zu einem späteren Zeitpunkt t₃ (bei der Temperatur T₃, die von T₂ abweichen kann), ist wiederum ein weiterer Teil der Signal-Oligonukleotide der Teststelle verdrängt (Fig. 2d).

Nach der Hybridisierung von Target-Oligonukleotiden und Signal-Oligonukleotiden wird in einer zweiten und eventuell eine Reihe weiterer Messungen in Abhängigkeit von Hybridisierungszeit und -temperatur (z.B. einer zweiten / weiteren cyclovoltammetrischen oder chronocoulometrischen Messung) der Anteil der verbliebenen oberflächenimmobilisierten Signal-Nukleinsäureoligomere bestimmt. Die Differenz aus Referenzmessung und zweiter/weiterer Messung je Test-Site ist proportional zur Anzahl der ursprünglich in der Untersuchungslösung für das jeweilige Test-Site vorhandenen Target-Oligonukleotide. In Figur 2e ist der Verlauf einer solchen Detektion schematisch dargestellt.

Alternativ kann die Referenzmessung weggelassen werden, z. B. wenn die Größe des Referenzsignals vorher (z.B. durch vorausgegangene Messungen etc.) hinlänglich genau bekannt ist oder wenn eine Referenz-Test-Site wie in Fig. 3 (iii) dargestellt, auf dem Chip vorhanden ist oder wenn nach Targetzugabe mehrere Messungen zeit-und/oder temperaturabhängig durchgeführt werden.

### a) Kovalente Anbindung von Sonden-Oligonukleotiden an (eine) einzeln adressierbare Gold-Elektrode(n), Hybridisierung mit redoxgelabelten Signal-Nukleinsäureoligomeren, Zugabe der Target-Oligonukleotide und cyclovoltammetrische Detektion der Verdrängung der Signal-Nukleinsäureoligomere durch die Target-Nukleinsäureoligomere:

Die n Nukleotide (nt) lange Sonden-Nukleinsäure (DNA, RNA oder PNA, z.B. ein 20 Nukleotide langes Oligo) (Fig. 1, III) ist in der Nähe eines ihrer Enden (3'- oder 5'-Ende) direkt oder über einen (beliebigen) Spacer mit einer reaktiven Gruppe zur kovalenten Verankerung an der Oberfläche versehen, z.B. als 3'-Thiol-modifiziertes Sonden-Oligonukleotid, bei dem die endständige Thiolmodifikation als reaktive Gruppe zur Anbindung an Goldelektroden dient. Als Spacer eignen sich auch mehrere (2 bis 40) "unspezifische" Basen, d.h. eine Spacer-Basenabfolge, die zu keinem Sequenzbereich des Signal-Nukleinsäureoligomers oder des Target-Nukleinsäureoligomers komplementär oder weitgehend komplementär ist. Idealerweise ist diese "unspezifische" Spacerbasenabfolge des Sonden-Nukleinsäureoligomers über seine gesamte Länge nicht hybridisierend zu beliebigen Sequenzbereichen aller Arten von Signal-Nukleinsäureoligomeren oder aller Arten von Target-Nukleinsäureoligomeren oder auch zum Sonden-Nukleinsäureoligomer selbst (letzteres zur Vermeidung von Loops).

Weitere kovalente Verankerungsmöglichkeiten ergeben sich z.B. aus aminomodifiziertem Sonden-Oligonukleotid, das zur Verankerung an oberflächlich zur Carbonsäure aufoxidierten Glaskohlenstoffelektroden oder an Platinelektroden verwendet wird. Zusätzlich kann ein monofunktionaler Linker geeigneter Kettenlänge mit identischer reaktiver Gruppe bereit gestellt werden. Das so modifizierte Sonden-Nukleinsäureoligomer wird
(i) in Puffer (z.B. 50 - 500 mM Phosphat-Puffer, pH = 7, 1mM EDTA) gelöst mit der Oberfläche in Kontakt gebracht und dort über die reaktive Gruppe des Sonden-Nukleinsäureoligomers an die - gegebenenfalls entsprechend derivatisierte - Oberfläche angebunden oder
(ii) in Gegenwart eines monofunktionalen Linkers in Puffer (z.B. 100 mM Phosphat-Puffer, pH = 7, 1mM EDTA, 0,1 - 1 M NaCl) gelöst mit der Oberfläche in Kontakt gebracht und dort über die reaktive Gruppe des Sonden-Nukleinsäureoligomers gemeinsam mit dem monofunktionalen Linker an die - gegebenenfalls entsprechend derivatisierte - Oberfläche angebunden, wobei darauf geachtet wird, dass genügend monofunktionaler Linker geeigneter Kettenlänge zugesetzt wird (etwa 0,1 bis 10 facher oder sogar 100 facher Überschuss), um zwischen den einzelnen Sonden-Oligonukleotiden genügend Freiraum für eine Hybridisierung mit den redoxgelabelten Signal-Nukleinsäureoligomeren bzw. dem Target-Oligonukleotid zur Verfügung zu stellen oder
(iii) in Puffer (z.B. 10 - 350 mM Phosphat-Puffer, pH = 7, 1mM EDTA) gelöst mit der Oberfläche in Kontakt gebracht und dort über die reaktive Gruppe des Sonden-Nukleinsäureoligomers an die - gegebenenfalls entsprechend derivatisierte - Oberfläche angebunden. Anschließend wird die so modifizierte Oberfläche mit monofunktionalen Linker in Lösung (z.B. Alkanthiole oder ω-Hydroxy-Alkanthiole in Phosphat-Puffer/EtOH-Mischungen bei Thiol-modifizierten Sonden-Oligonukleotiden) in Kontakt gebracht, wobei der monofunktionale Linker über seine reaktive Gruppe an die - gegebenenfalls entsprechend derivatisierte - Oberfläche anbindet.

Die so modifizierte Oberfläche wird, nach entsprechenden Waschschritten, mit redoxgelabelten Signal-Nukleinsäureoligomeren (Fig. 1, IV) in Kontakt gebracht. Als mit Redoxlabel markierte Signal-Nukleinsäureoligomer kann z.B. ein- oder mehrfach Ferrocencarbonsäure-modifiziertes 40 Basen langes Signal-Oligonukleotid verwendet werden, dessen Sequenz 5'-terminal zur Sequenz des Sonden-Oligonukleotids komplementär oder weitgehend komplementär ist und dessen komplette Sequenz komplementär oder weitgehend komplementär zur Target-Oligonukleotid-Sequenz ist. Dabei wird darauf geachtet, dass deutlich mehr markierte Signal-Nukleinsäureoligomere (mindestens 1.1facher molarer Überschuss) zugegeben werden als über die Sonden-Nukleinsäureoligomere an der Oberfläche gebunden werden können. Nach Hybridisierung der Signal-Oligonukleotide an die Sonden-Oligonukleotide werden überschüssige, nicht hybridisierte Signal-Oligonukleotide durch Spülen mit Puffer (z.B. 350 mM Phosphat-Puffer, pH = 7, 1 mM EDTA) entfernt.

Das Inkontaktbringen der Signal-Oligonukleotide mit der mit Sonden-Oligonukleotiden derivatisierten Oberfläche kann z.B. durch Eintauchen der derivatisierten Oberfläche in die Signal-Oligonukleotid-Lösung oder durch Bespotten der derivatisierten Oberfläche mittels geeigneter Verfahren (Pipette, Mikrodosiersysteme etc.) erfolgen. Bei der Realisierung mehrerer Test-Sites für verschiedene Target-Oligonukleotide erfolgt die Immobilisierung der Sonden-Oligonukleotid-Arten (für je ein Target) in räumlich voneinander getrennten Bereichen. Auch hier kann das Inkontaktbringen der Signal-Oligonukleotide mit der derivatisierten Oberfläche durch Eintauchen der derivatisierten Oberflächen (also aller räumlich getrennter Bereiche mit den jeweiligen Sonden-Oligonukleotiden gleichzeitig) in die Lösung mit den Signal-Oligonukleotid-Arten erfolgen, wenn die Sequenzunterschiede bei den Signal-Oligonukleotid-Arten groß genug sind, um die jeweiligen Signal-Oligonukleotide ausschließlich oder zumindest größtenteils ausschließlich an die zugehörigen Sonden-Oligonukleotiden zu hybridisieren. Ist dies nicht gewährleistbar, wird das "Bespotten" der einzelnen räumlich getrennten derivatisierten Oberflächen mit der jeweils zugehörigen Signal-Oligonukleotide-Lösung bevorzugt. Letztere Methode ist in jedem Fall anwendbar.

Das Detektionslabel am Signal-Nukleinsäureoligomer wird durch ein geeignetes Verfahren detektiert, z.B. durch Cyclovoltammetrie oder Chronocoulometrie im Fall der mit Ferrocen Redox-Label-markierten Signal-Oligonukleotide (Referenzmessung). Anschließend wird das gelöste Target zugegeben und die Messung zur Detektion der Detektionslabel mit dem geeigneten Verfahren (zeit-und/oder temperaturabhängig mehrfach) wiederholt (z.B. erneute cyclovoltammetrische oder chronocoulometrische Messung im Fall der mit Ferrocen Redox-Label markierten Signal-Oligonukleotide).

Die Hybridisierung kann unter geeigneten, dem Fachmann bekannten Bedingungen durchgeführt werden (beliebige, frei wählbare Stringenzbedingungen der Parameter Potential/Temperatur/Salz/chaotrope Salze etc. für die Hybridisierung). Der Unterschied zwischen Referenzmessung und Messung(en) nach Targetzugabe ist proportional zur Anzahl der Hybride aus Signal-Nukleinsäureoligomeren und passenden Target-Nukleinsäureoligomeren, die die modifizierte Oberfläche zur Volumenphase hin verlassen.

Das Verfahren kann für eine Target-Art, also eine bestimmte Target-Oligonukleotid-Art mit bekannter Sequenz, an einer Elektrode oder für mehrere Target-Arten, also verschiedene Target-Oligonukleotid-Arten, an einzeln adressierbaren Elektroden eines Elektrodenarrays, das bei komplexeren Arrays z.B. über CMOS-Technologie ansteuer- und auslesbar ist, angewendet werden.

### b) Indirekte Anbindung von Sonden-Oligonukleotiden an Glasfasern, mit Fluorophor markierte Signal-Nukleinsäureoligomere, Target-Oligonukleotide und Fluoreszenzdetektion der Verdrängung der Signal-Nukleinsäureoligomere durch die Target-Nukleinsäureoligomere:

Die n Nukleotide lange Sonden-Nukleinsäure (DNA, RNA oder PNA) (z.B. ein 20 Nukleotide langes Oligo), ist in der Nähe eines ihrer Enden (3'- oder 5'-Ende) direkt oder über einen (beliebigen) Spacer mit einer reaktiven Gruppe zur kovalenten Verankerung an der Oberfläche versehen, z.B. ein Carboxy-modifiziertes Sonden-Oligonukleotid zur Anbindung an aminomodifiziertes silanisiertes Glas oder Silizium (z.B. an (3-Aminopropyl)-triethoxysilan modifiziertes Glas oder Silizium). Weitere kovalente Verankerungsmöglichkeiten ergeben sich z.B. aus aminomodifiziertem Sonden-Oligonukleotid, das zur Verankerung an mit Carbonsäure derivatisierten und auf Glas oder Silizium immobilisierten Dextranpolymeren verwendet wird, wobei bei dieser Ausführungsform die Dicke der Schicht aus Dextranpolymer mit angebundenen Sonden-Oligonukleotiden mit dem Fachmann bekannten Methoden über die Dextranpolymerzusammensetzung, Dextranankergruppen an der Glasoberfläche, Ankergruppen des Dextrans für die Immobilisierung am Glas, Inkubationsdauer am Glas etc. variiert werden kann. In einer bevorzugten Ausführungsform wird die Dicke der Dextran/Sonden-Oligonukleotidschicht so gewählt, dass sie in etwa der Eindringtiefe des evaneszenten Feldes des Lichts zur Anregung der Fluorophore entspricht (ca. 50 nm bis ca. 500 nm, in Abhängigkeit davon, ob sich auf dem Glas zur Erhöhung der Eindringtiefe des evaneszenten Feldes eine Metallisierungsschicht befindet).

Das so modifizierte Sonden-Nukleinsäureoligomer wird in Gegenwart von EDC und sNHS (jeweils ca. 40facher molarer Überschuss in Bezug auf das Sonden-Oligonukleotid) in Puffer (z.B. 50 - 500 mM Phosphat-Puffer, pH = 7, 1mM EDTA) gelöst, mit der modifizierten Glas- (bzw. Silizium) Oberfläche in Kontakt gebracht und dort über die reaktive Gruppe des Sonden-Nukleinsäureoligomers an die Oberfläche angebunden (gegebenenfalls werden vorher nichtfunktionelle Oberflächenbindungsstellen mit einem geeigneten Blocking-Reagenz behandelt).

Die so modifizierte Oberfläche wird, nach geeigneten Waschschritten, mit Fluorophor-markierten Signal-Nukleinsäureoligomeren, analog wie unter Ausführungsform a) beschrieben, in Kontakt gebracht. Als mit Fluorophor markierte Signal-Nukleinsäureoligomere können z.B. Fluorescein-modifizierte NukleinsäureOligomere (z.B. der Basenlänge 40) verwendet werden, deren Sequenz 5'-terminal zur Sequenz des Sonden-Oligonukleotids komplementär oder weitgehend komplementär ist und dessen komplette Sequenz komplementär oder weitgehend komplementär zur Target-Oligonukleotid-Sequenz ist. Dabei wird darauf geachtet, dass deutlich mehr markierte Signal-Nukleinsäureoligomere (mindestens 1.1facher molarer Überschuss) zugegeben werden als über die Sonden-Nukleinsäureoligomere an der Oberfläche gebunden werden können. Nach Hybridisierung der Signal-Oligonukleotide an die Sonden-Oligonukleotide werden überschüssige, nicht hybridisierte Signal-Oligonukleotide durch Spülen mit Puffer (z.B. 350 mM Phosphat-Puffer, pH = 7, 1 mM EDTA) entfernt.

Das Detektionslabel am Signal-Oligonukleotid wird durch ein geeignetes Verfahren detektiert, z.B. durch Total Internal Reflection Fluorescence (TIRF) im Fall der mit Fluorophor-Label markierten Signal-Nukleinsäureoligomere (Referenzmessung). Anschließend wird die Untersuchungslösung zugegeben und potentielle Hybridisierungsereignisse werden unter geeigneten, dem Fachmann bekannten Bedingungen ermöglicht (beliebige, frei wählbare Stringenzbedingungen der Parameter Potential/Temperatur/Salz/chaotrope Salze etc. für die Hybridisierung).

Daraufhin wird die Messung zur Detektion des Detektionslabels mit dem geeigneten Verfahren (mehrfach zeit- und/oder temperaturabhängig) wiederholt (z.B. erneute TIRF-Messung im Fall der mit Fluorescein markierten Signal-Nukleinsäureoligomeren). Der Unterschied zwischen Referenzmessung und den Messungen nach Targetzugabe ist proportional zur Anzahl der Hybridisierungsereignisse zwischen Signal-Nukleinsäureoligomer und passendem Target-Nukleinsäureoligomer in der Untersuchungslösung, die die Oberfläche als Hybrid verlassen. Bei der Detektion durch Bestimmung der TIRF ist eine Abnahme des Fluoreszenzsignals zu erwarten.

Das Verfahren kann für eine Target-Art, also eine bestimmte Target-Oligonukleotid-Art mit bekannter Sequenz, z.B. an einer Glasfaser oder für mehrere Target-Arten, also verschiedene Target-Oligonukleotid-Arten, z.B. an einzeln adressierbaren Glasfasern eines Glasfaserbündels angewendet werden.

### c) Ausführungsformen des Verdrängungsassays und möglicher Referenz-Teststellen (Fig. 3):

Als Target-Nukleinsäureoligomer (Sequenz 5' nach 3') wird der Einzelstrang bezeichnet, der über das Assay erfasst werden soll. Es umfasst einen Sequenzabschnitt, der bevorzugt zur Identifizierung/Anbindung an die Sonde bzw. das Signal-Oligo und zur Diskriminierung von anderen Targets dient ("Displace"-Bereich). Unmittelbar oder mittelbar angrenzende Sequenzbereiche "up- oder downstream" davon können herangezogen werden, um eine Hybridisierung an den Andock-Abschnitt des Sonden bzw. Signal-Nukleinsäureoligiomers zu ermöglichen.

In Fig. 3B werden zwei prinzipielle Möglichkeiten (i) und (ii) dargestellt, das Hybrid aus an der Oberfläche immobilisiertem Sonden- und anhybridisiertem Signal-Nukleinsäureoligomer mit nicht hybridisiertem Andock-Bereich für das Target zu realisieren. Fig. 3B (i) zeigt den Fall, in dem der Andock-Bereich erfindungsgemäß am Sonden-Nukleinsäureoligomer lokalisiert ist. Das 5'-Ende des Sonden-Nukleinsäureoligomers steht deutlich über das 3'-Ende des Signal-Nukleinsäureoligomers hinaus und stellt in dem überstehenden Abschnitt den Andock-Bereich zur Verfügung. Zum Vergleich ist in Fig. 3B (ii) dargestellt, dass der Andock-Bereich auch auf dem Signal-Nukleinsäureoligomer lokalisiert sein kann.

Das 3'-Ende des Signal-Nukleinsäureoligomers steht deutlich über das 5'-Ende des Sonden-Nukleinsäureoligomers hinaus und stellt in dem überstehenden Abschnitt den Andock-Bereich zur Verfügung.

Neben den in Fig. 3B (i) und (ii) dargestellten Hybridisierungsbereichen zwischen Sonde und Signal-Oligo kann auch jede andere Hybridstruktur herangezogen werden. So kann ausgehend von der in (i) dargestellten Struktur das Signal-Oligo - bei entsprechend geänderter Sequenz - in Richtung 3' oder 5' der Sonde verschoben sein bis es im Extremfall am 5'-terminalen Ende sitzt (oder darüber hinaus geht). Eine solche Situation ist in Fig. 3B (iv) dargestellt. In diesem Fall sind auf dem Sonden-Nukleinsäureoligomer zwei Andock-Beriche lokalisiert, einer beginnend am 5'-Ende des Sonden-Nukleinsäureoligomers in Richtung des 3'-Endes des Signal-Oligos und ein zweiter beginnend am 3'-Ende des Sonden-Nukleinsäureoligomers in Richtung des 5'-Endes des Signal-Oligos. Fig. 3B (v) zeigt den analogen Fall für zwei Andock-Abschnitte auf dem Signal-Nukleinsäureoligomer.

Bei (i) liegt also der Andock-Abschnitt (nicht hybridisierter Bereich im Addukt aus Sonden- und Signal-Nukleinsäureoligomer) auf dem Sonden-Nukleinsäureoligomer. An diesen Andock-Abschnitt hybridisiert nach Probenzugabe - bei entsprechender Basensequenz dieses Abschnitts - ein Sequenzbereich 103 des Targets. Gleiches gilt analog für (ii), so dass hier das 3'-Ende des Signal-Oligos z.B. mit dem 5'-Ende der Sonde hybridisiert oder sogar weiter downstream von der Sondensequenz hybridisiert. Im Falle (ii) liegt der Andock-Abschnitt (nicht hybridisierter Bereich im Addukt aus Sonden- und Signal-Nukleinsäureoligomer) auf dem Signal-Nukleinsäureoligomer. An diesen Andock-Abschnitt hybridisiert nach Probenzugabe - bei entsprechender Basensequenz dieses Abschnitts - ein Sequenzbereich 101 des Targets.

Neben der eigentlichen Teststelle nach einer der Variationen in (i) und (ii) kann eine Referenz-Test-Site (iii) realisiert werden (z.B. als on-chip Referenz): für eine beliebige Sequenz des Hybrids aus Sonden- und Signalnukleinsäureoligomeren in den Fällen (i) und (ii) werden dazu die so genannten "inversen" Sequenzen des Hybrids zur Konstruktion einer Sonde mit zugehörigem Signal-Oligonukleotid gleicher Basenlänge verwendet, die aber keine Andockstelle und eine umgekehrte Basenabfolge aufweist. Die Schmelzkurve des inversen Hybrids weist theoretisch in etwa den gleichen Verlauf (Signal vs. Temperatur) auf wie der hybridisierte Anteil in (i) bzw. (ii). Als inverse Sequenz wird die Basenabfolge, die normalerweise in 5' nach 3' angegeben ist, beibehalten, aber in inverser Richtung (3' nach 5'). Beispiesweise ist zur Sequenz 5'-GTTCAAAG-3' ist die Sequenz 3'-GTTCAAAG-5' (und somit 5'-GAAACTTG-3') invers.

### Beispiel 1: Darstellung der N-Hydroxysuccinimid-Aktivester der Redox- (oder Fluorophor-) Label

1 mmol des jeweiligen Carbonsäurederivates eines Fluorophors (z.B. Fluorescein) bzw. einer redoxaktiven Substanz (z.B. Ferrocen) und 1.1 mmol N-Hydroxysuccinimid werden in 15 ml wasserfreiem Dioxan gelöst. 1.1 mmol Carbodiimid (gelöst in 3 ml wasserfreiem Dioxan) werden unter Eiskühlung zum Carbonsäurederivat getropft. Das Reaktionsgemisch wird 16 h bei RT gerührt, der gebildete Niederschlag abfiltriert und das Lösungsmittel abgezogen. Der Rückstand wird durch Kieselgelchromatographie aufgereinigt (Merck Kieselgel 60, Laufmittel: Dichlormethan/Ethylacetat/Heptan-Mischungen).

### Beispiel 2: Darstellung der aminomodifizierten Oligonukleotide zur Kopplung der Aktivesterlabel aus Bsp. 1 bzw. von thiolmodifizierten Oligonukleotiden zur Verankerung auf Gold als Sonden-Nukleinsäureoligomere

Die Synthese der Oligonukleotide erfolgt in einem automatischen Oligonukleotid-Synthesizer (Expedite 8909; ABI 384 DNA/RNA-Synthesizer) gemäß der vom Hersteller empfohlenen Syntheseprotokolle für eine 1.0 µmol Synthese. Die Synthese der Signal-Nukleinsäureoligomere erfolgt standardmäßig n A-CPG als Trägermaterial. Modifikationen an der 5'-Position der Oligonukleotide erfolgen mit einem auf 5 Minuten verlängerten Kopplungsschritt. Der Amino-Modifier C2 dT (Glen Research 10-1037) wird in die Sequenzen mit dem jeweiligen Standardprotokoll eingebaut.

Die Darstellung von 3'-dithiolmodifizierten Sonden-Oligonukleotiden erfolgt auf 5-Hydroxy-1,2-Dithian-4-O-Dimethoxytrityl modifiziertem CPG-Träger, weitere Dithiol-Modifikationen erfolgen mittels 1,2-Dithian-4-O-Dimethoxytrityl-5-[(2-cyanoethyl)-N,N-diisopropyl)]-phosphoramidit (DTPA, Glen Research 10-1937) analog zu Standardprotokollen, wobei die Oxidationsschritte mit einer 0.02 M lodlösung durchgeführt werden, um eine oxidative Spaltung der Disulfidbrücke(n) zu vermeiden. Die Kopplungseffizienzen werden während der Synthese online über die DMT-Kationen-Konzentration photometrisch bzw. konduktometrisch bestimmt.

Die Oligonukleotide werden mit konzentriertem Ammoniak (30%) bei 37 °C über einen Zeitraum von 16 h entschützt. Die Reinigung der Oligonukleotide erfolgt mittels RP-HPL Chromatographie nach Standardprotokollen (Laufmittel: 0.1 M Triethylammoniumacetat-Puffer, Acetonitril), die Charakterisierung mittels MALDI-TOF MS.

### Beispiel 3: Umsetzung der aminomodifizierten Oligonukleotide (Bsp. 2) mit den N-Hydroxy-Aktivestern (Bsp. 1)

Die aminomodifizierten Oligonukleotide werden in 0.1 M Boratpuffer (pH 8.5) gelöst und mit den in DMSO gelösten N-Hydroxysuccinimid-Aktivestern gemäß dem Protokoll von Molecular Probes (Labeling Amine-Modified Oligonucleotides) umgesetzt. Die Reinigung der Oligonukleotide erfolgt mittels RP-HPL Chromatographie nach Standardprotokollen (Laufmittel: 0.1 M Triethylammoniumacetat-Puffer, Acetonitril), die Charakterisierung mittels MALDI-TOF MS.

### Beispiel 4: Herstellung der Sonden- und Signal-Oligonukleotide für ein HPV_6 Assay

Zur Detektion von HPV_6 in Form eines Verdrängungsassays gemäß einer Ausführungsform der vorliegenden Erfindung wurden entsprechend den Bsp. 1 bis 3 folgenden Sequenzen synthetisiert:
Sonde HPV_6:
   5'-cgta T ctacatcttccac T tacaccaa CATATTTATT-(S-S)₃-3'
Signal-Oligo HPV_6:
   5'-(Fc)₄-ttggtgtatgtggaagatgtagttacg_gatgtacataatgtcatgttggtactgcg-3'
Sonde HPV_6_Ref:
   5'-ttggtgta A gtggaagatgtag A tacg TCAATTTTTTT-(S-S)₃-3'
Signal-Oligo*HPV_6_Ref
   5'-(Fc)₄-cgtaactacatcttccacatacaccaa

Hierbei stehen in den Sonden die Großbuchstaben für die "unspezifische" Spacer-Basenabfolge. Basen in Großbuchstaben und Unterstreichung sind nicht komplementär zum Signal-Oligonukleotid. (S-S)₃ steht für drei DTPA-Einheiten (siehe Bsp. 2) mit insgesamt sechs Thiofunktionen zur Immobilisierung der Sonde auf der Goldelektrode. Bei den Signal-Oligonukleotiden steht (Fc)₄ für vier kovalent an das Signal-Olignukleotid angebundene Ferrocen-Label. Beim Signal-Oligo HPV-6 ist der Bereich zur Hybridisierung an die Sonde und der Andockbereich durch einen Unterstrich getrennt (Sequenzabschnitt für die Sonden- Hybridisierung-_Sequenzabschnitt des Andock-Bereichs).

### Beispiel 5: Herstellung der Oligonukleotid-Elektroden "HPV_6" und "HPV_6_Ref"

Das Trägermaterial für die kovalente Anbindung der Doppelstrang-Oligonukleotide bildet eine Gold-Mikroelektrode innerhalb eines Elektrodenarrays. Die Goldoberfläche wir unmittelbar vor der Inkubation mit den Sonden (Sonde HPV_6 bzw. Sonde HPV_6_Ref, vgl. Bsp. 2 und 4) in 0,5 M H₂SO₄ als Elektrolyt durch cyclovoltammetrische Messungen im Bereich von 0 bis 1,35 V vs. Ag/AgCl von Oberflächenverunreinigungen befreit. Nach diesem allgemein als Elektropolieren bezeichneten Vorgang wird das Elektrodenarray mit Wasser und anschließend mit Ethanol gepült, restliche Ethanolspuren weren im Ar-Strom entfernt.

Zur Inkubation wird Sonde HPV_6 bzw. Sonde HPV_6_Ref als 5x10⁻⁵ molare Lösung in 500 mM Na-Phosphat-Puffer, pH 7, gelöst und über Mikro-Dosiersysteme auf je eine Teststelle des Elektrodenarrays gebracht, so dass die Gold-Oberfläche eines Test-Sites komplett benetzt ist, und für 2 h inkubiert. Das dazu eingesetzte contact- oder non-contact-Printing ist in der WO 2004/082814 A2 ausführlich beschrieben, auf die in diesem Zusammenhang Bezug genommen wird. Während dieser Reaktionszeit werden die Disulfide der DTPA-Einheiten (vgl. Bsp. 2 und 4) via Chemisorption an der Goloberfläche verankert.

Anschließend wird die so modifizierte Oberfläche mit Puffer (500 mM Na-Phosphat, pH=7) gespült und nachfolgend mit 6-Hydroxy-Hexanthiol in 250 mM Phosphat-Puffer, pH=7, mit 1% Ethanol für 6 h inkubiert, um freies Gold zu passivieren (Anbindung der Alkanthiole an Gold via Chemisorption).

Nach erfolgter Alkanthiolinkubation wird das Elektrodenarray erneut gespült (mit 250mM Na-phosphat-Puffer, pH = 7, danach mit Ethanol), im Ar-Strom trockengeblasen und danach mit einer Lösung der Signal-Oligonukleotide (Signal-Oligo HPV_G und Signal-Oligo HPV_6_Ref, vgl. Bsp. 2 und 4) 50 nM in Na-Phosphat-Puffer, pH = 7) für ca. 20 min inkubiert, erneut gespült (350 mM Na-Phosphat-Puffer, pH = 7) und im Ar-Strom getrocknet.

### Beispiel 6: Verdrängungsassy für HPV-6 mit Temperaturgradienten.

Der in Bsp. 5 erhaltene Chip wird in Elektrolyt (250 mM Na₂SO₄) cyclovoltammetrisch vermessen (Potentiostat Autolab PGSTAT 12 der Fa. Metrohm, Potentialbereich 0 bis 0,5 V vs. Ag/AgCl, Scan Rate 500 mV/s, Raumtemperatur, hier 26°C). Bei dieser Messung wird eine Strom-Spannungskurve erhalten, wobei der Peakstrom des erhaltenen Cyclovoltagramms proportional zur Anzahl der (oxidierbaren) Ferrocene und damit proportional zur Anzahl der Signal-Oligonukleotide und damit proportional zur Anzahl der Hybride aus Signal- und Sonden-Oligonukleotid ist. Die so erhaltene Messung dient als Referenzwertbestimmung (Referenzmessung).

Anschließend wird der Elektrolyt und damit die Elektroden mit 2°C/min aufgeheizt und die Messungen werden unter ansonsten identischen Bedingungen mehrfach wiederholt. In Fig. 4A ist das Messergebnis dieser Vorgehensweise, normiert auf den Referenzwert wiedergegeben. Der Kurvenverlauf entspricht im Wesentlichen einer Schmelzkurve der Hybride aus Sonde und Signal-Oligonukleotid.

Die gleiche Messprozedur wird anschließend wiederholt, wobei aber nach der Referenzmessung ein HPV_6-Target-Nukleinsäureoligomer zum Elektrolyten gegeben wird (asymmetrisches PCR-Produkt in Wasser, 130 Basen lang mit einem Bereich, der komplementär zur Signal-Oligonukleotid-Sequenz ist). Das Target lag bei der Messung in Fig. 4B in einer Konzentration von ca. 10nM vor.

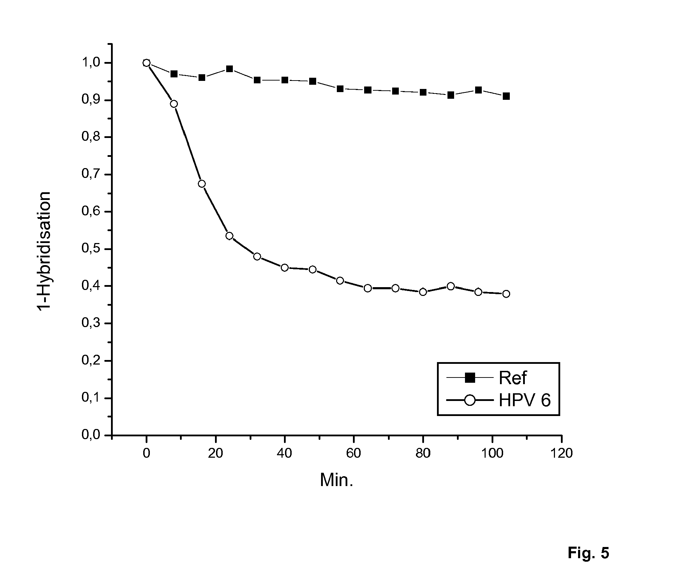

Statt mit Temperaturgradient können die Messungen mit und ohne Target auch wie folgt durchgeführt werden. Zunächst wird eine Referenzmessung durchgeführt, anschließend wird die Hybridisierung zwischen Target und Signal-Oligonukleotid bei geeigneter Temperatur durch wiederholtes Messen verfolgt. Im Falle der in Fig. 5 dargestellten Messungen erfolgte die Referenzmessung bei Raumtemperatur, die weiteren Messungen in Abhängigkeit von der Zeit bei 38°C. Ansonsten entsprachen die Bedingungen den oben im Zusammenhang mit den in Fig. 4 dargestellten Messungen geschilderten Bedingungen.

## Patentansprüche

1. Verfahren zur Detektion von Nukleinsäureoligomer-Hybridisierungsereignissen umfassend die Schritte
a) Bereitstellen einer modifizierten Oberfläche, wobei die Modifikation in der Anbindung wenigstens einer Art von Sonden-Nukleinsäureoligomeren besteht,
b) Bereitstellen wenigstens einer Art von Signal-Nukleinsäureoligomeren, wobei die Signal-Nukleinsäureoligomere mit zumindest einem Detektionslabel modifiziert sind und die Signal-Nukleinsäureoligomere einen zu den Sonden-Nukleinsäureoligomeren komplementären oder weitgehend komplementären Abschnitt besitzen,
c) Bereitstellen einer Probe mit Target-Nukleinsäureoligomeren,
d) Inkontaktbringen einer definierten Menge der Signal-Nukleinsäureoligomere mit der modifizierten Oberfläche,
e) Inkontaktbringen der Probe und der darin enthaltenen Target-Nukleinsäureoligomere mit der modifizierten Oberfläche,
f) Detektion der Signal-Nukleinsäureoligomere,
g) Vergleich der in Schritt f) erhaltenen Werte mit Referenzwerten,
**dadurch gekennzeichnet, dass** die Signal-Nukleinsäureoligomere eine größere Anzahl an Basen besitzen als die Sonden-Nukleinsäureoligomere und die Signal-Nukleinsäureoligomere zumindest einen Andockabschnitt aufweisen, wobei der Andockabschnitt keine zu einem Abschnitt der Sonden-Nukleinsäureoligomere komplementäre oder weitgehend komplementäre Struktur aufweist und wobei die Target-Nukleinsäureoligomere einen zu dem Andockabschnitt komplementären oder weitgehend komplementären Abschnitt besitzen.

2. Verfahren nach Anspruch 1, wobei sich die Zahl der Basen von Sonden-Nukleinsäureoligomeren und Signal-Nukleinsäureoligomeren um 6 bis 80, insbesondere um 9 bis 60, besonders bevorzugt um 10 bis 40 unterscheidet.

3. Verfahren nach zumindest einem der Ansprüche 1 und 2, wobei nach Schritt d) und vor Schritt e) der Schritt
d₁) Detektion der Signal-Nukleinsäureoligomere zur Bestimmung von Referenzwerten
durchgeführt wird und in Schritt g) die in Schritt f) erhaltenen Werte mit den in Schritt d₁) erhaltenen Referenzwerten verglichen werden.

4. Verfahren nach zumindest einem der Ansprüche 1 bis 3, wobei die Signal-Nukleinsäureoligomere 10 bis 200 Nukleinsäuren, insbesondere 20 bis 100 Nukleinsäuren, besonders bevorzugt 25 bis 70 Nukleinsäuren umfassen.

5. Verfahren nach zumindest einem der Ansprüche 1 bis 4, wobei Signal-Nukleinsäureoligomere und Sonden-Nukleinsäureoligomere verwendet werden, die in den miteinander hybridisierenden Bereich ihrer Sequenz zumindest ein nicht-komplementäres Basenpaar aufweisen.

6. Verfahren nach zumindest einem der Ansprüche 1 bis 5, wobei die Detektion der Signal-Nukleinsäureoligomere mehrfach wiederholt wird.

7. Verfahren nach Anspruch 6, wobei die erste Detektion der Signal-Nukleinsäureoligomere in einer Puffer-Lösung vor dem Inkontaktbringen der Probe mit der modifizierten Oberfläche erfolgt.

8. Verfahren nach zumindest einem der Ansprüche 6 und 7, wobei die erste Detektion der Signal-Nukleinsäureoligomere nach dem Inkontaktbringen der Probe und der darin enthaltenen Target-Nukleinsäureoligomere mit der modifizierten Oberfläche sofort nach dem Inkontaktbringen der Probe mit der modifizierten Oberfläche erfolgt und anschließend über einen Zeitraum von zumindest 40 Min. mehrfach wiederholt wird.

9. Verfahren nach Anspruch 8, wobei die Detektion der Signal-Nukleinsäureoligomere über einen Zeitraum von zumindest 20 Min., insbesondere über einen Zeitraum von zumindest 10 Min., besonders bevorzugt über einen Zeitraum von zumindest 5 Min. mehrfach wiederholt wird.

10. Verfahren nach zumindest einem der Ansprüche 6 bis 9, wobei während der mehrfachen Wiederholung der Detektion der Signal-Nukleinsäureoligomere die Temperatur im Bereich der modifizierten Oberfläche ausgehend von Raumtemperatur verändert wird.

11. Verfahren nach Anspruch 10, wobei die Temperatur im Bereich der modifizierten Oberfläche um 1°C pro Min. bis 10°C pro Min., insbesondere um 2°C pro Min. über die Raumtemperatur erhöht wird.

12. Verfahren nach zumindest einem der Ansprüche 1 bis 11, wobei als Schritte a) und b) der Schritt
ab) Bereitstellen einer modifizierten Oberfläche, wobei die Modifikation in der Anbindung wenigstens einer Art von Sonden-Nukleinsäureoligomeren besteht, und wobei wenigstens eine Art von zumindest mit einem Detektionslabel modifizierten Signal-Nukleinsäureoligomeren an die Sonden-Nukleinsäureoligomere hybridisiert vorliegt
durchgeführt wird und der Schritt d) entfällt.

13. Modifizierte Oberfläche, wobei die Modifikation in der Anbindung wenigstens einer Art von Sonden-Nukleinsäureoligomeren besteht, und wobei wenigstens eine Art von zumindest mit einem Detektionslabel modifizierten Signal-Nukleinsäureoligomeren an die Sonden-Nukleinsäureoligomere hybridisiert vorliegt, wobei die Signal-Nukleinsäureoligomere eine größere Anzahl an Basen besitzen als die Sonden-Nukleinsäureoligomere und die Signal-Nukleinsäureoligomere zumindest einen Andockabschnitt aufweisen, wobei der Andockabschnitt keine zu einem Abschnitt der Sonden-Nukleinsäureoligomere komplementäre oder weitgehend komplementäre Struktur aufweist.

14. Modifizierte Oberfläche nach Anspruch 13, wobei Signal-Nukleinsäureoligomere und Sonden-Nukleinsäureoligomere verwendet werden, die in den miteinander hybridisierenden Bereich ihrer Sequenz zumindest ein nicht-komplementäres Basenpaar aufweisen.

15. Kit zur Durchführung eines Verfahrens nach zumindest einem der Ansprüche 1 bis 12 umfassend eine modifizierte Oberfläche wie in zumindest einem der Ansprüche 1 bis 14 definiert und eine effektive Menge an Signal-Nukleinsäureoligomeren wie in zumindest einem der Ansprüche 1 bis 14 definiert.
